# EUROPEAN PATENT APPLICATION

(11) **EP 4 183 888 A1**
(43) Date of publication of application: **24.05.2023**
(21) Application number: 21383049.0
(22) Date of filing: 22.11.2021
(51) Int. Cl.: C12Q 1/6876, C12Q 1/6883

(54) **MIRNA SIGNATURE FOR IDENTIFICATION OF THE RECEPTIVE ENDOMETRIUM**

(71) Applicant: Asociación Centro de Investigación Cooperativa en Biociencias - CIC bioGUNE, 48160 Derio, Vizcaya (ES); Universidad del País Vasco/Euskal Herriko Unibertsitatea, 48940 Leoia, Bikaia (ES); Administración General de la Comunidad Autónoma de Euskadi, 01010 Vitoria-Gasteiz (ES); REPRODALIA, S.L., 48013 Bilbao (ES)
(72) Inventor: IBÁÑEZ PÉREZ, Jone, 48940 Leioa - Bizkaia (ES); FALCÓN PÉREZ, Juan Manuel, 48160 Derio - Bizkaia (ES); MATORRAS WEINIG, Roberto, 48903 Barakaldo - Bizkaia (ES)
(74) Representative: ABG Intellectual Property Law, S.L.

(57) **Abstract**

The present invention relates to methods and kits for determining endometrial receptivity in a female subject and methods for selecting a female subject as a candidate to receive a therapy to increase or restore endometrial receptivity based on miRNA analysis of samples from a female subject. The method analyses endometrial fluid samples comprising extracellular vesicles. The miR analysed are hsa-miR-24-3p, hsa-miR-200b-3p, hsa-miR-148b-3p and hsa-miR-99b-5p.

SEQUENCE LISTING

<110>  ASOCIACIÓN CENTRO DE INVESTIGACIÓN COOPERATIVA EN
       BIOCIENCIAS-CIC bioGUNE
       UNIVERSIDAD DEL PAÍS VASCO/EUSKAL HERRIKO UNIBERTSITATEA
       ADMINISTRACIÓN GENERAL DE LA COMUNIDAD AUTÓNOMA DE EUSKADI
       REPRODALIA, S.L.

<120>  miRNA signature for identification of the receptive endometrium

<130>  P20921EP00

<160>  6

<170>  PatentIn version 3.5

<210>  1
<211>  22
<212>  RNA
<213>  Homo sapiens

<400>  1
uggcucaguu cagcaggaac ag                                              22


<210>  2
<211>  22
<212>  RNA
<213>  Homo sapiens

<400>  2
uaauacugcc ugguaaugau ga                                              22


<210>  3
<211>  22
<212>  RNA
<213>  Homo sapiens

<400>  3
ucagugcauc acagaacuuu gu                                              22


<210>  4
<211>  22
<212>  RNA
<213>  Homo sapiens

<400>  4
cacccguaga accgaccuug cg                                              22


<210>  5
<211>  23
<212>  RNA
<213>  Homo sapiens

<400>  5
uaauacugcc ggguaaugau gga                                             23


<210>  6
<211>  22

<212>  RNA
<213>  Homo sapiens

<400>  6
uauugcacuu gucccggccu gu                                                22

## Description

### FIELD OF THE INVENTION

The present invention relates to the field of assisted reproduction and, more in particular, to methods based on miRNA analysis for determining endometrial receptivity in a female subject and methods for selecting a female subject as a candidate to receive a therapy to increase or restore endometrial receptivity.

### BACKGROUND OF THE INVENTION

Embryo implantation is one of the most inefficient steps in assisted reproduction techniques. The implantation of an embryo is a complex process which requires a synchrony between the development of the embryo and the endometrium, but also an adequate embryo-endometrial cross talk. Increasing embryo implantation rates has become one of the greatest challenges in assisted reproduction techniques (ART) because only about 30% of the embryos that are transferred to the uterus successfully implant. In 2018, alone in Spain more than 40,000 women underwent assisted reproduction techniques with pregnancy rates by embryo transfer of about 35%. *In vitro* fertilization (IVF) involves several steps including ovarian stimulation, egg retrieval, sperm retrieval, fertilization and embryo transfer. One cycle of IVF can take about two to three weeks, and more than one cycle may be required for successful pregnancy.

Currently, endometrial ultrasound is the only universally accepted non-invasive tool to study the influence of the endometrium on embryo implantation. Endometrial tissues have been studied by histological, histochemical, and biochemical methods resulting in the identification of a number of molecules which are detectable in the endometrium in a cycle-dependent manner. Most studies on endometrial markers have been performed on endometrial cells using genomic or proteomic techniques.

The stage at which the endometrium is developmentally competent for embryo implantation is commonly referred to as "receptive endometrium", which attempts to embrace both the timing and status of this tissue for adequate receptivity. So far, it is not clear whether transferring an implantation-capable embryo into a "receptive endometrium" will result in a successful pregnancy. In fact, the timing at which the endometrium becomes receptive varies among different women or among different cycles of the same woman. In addition, controlled ovarian stimulation used in IVF cycles considerably alters the endometrium, resulting in both inadequate receptivity and/or changes in its timing.

Despite numerous researches focused on improving implantation rates, there is no reliable method for determining, prior embryo transfer, the receptive status of the endometrium and the correct timing for embryo transfer, which is fundamental for a successful embryo implantation and pregnancy. Thus, there is a need for a fast and meaningful method for determination of endometrial receptivity and for identification of patients who may require a therapy to increase endometrial receptivity.

### SUMMARY OF THE INVENTION

The authors of the present invention have found that the correct timing for receptivity of an endometrium can be reliably assessed by determining the level of miRNAs from a miRNA signature which is selected from the group consisting of a miRNA signature a) comprising hsa-miR-24-3p, hsa-miR-200b-3p and/or hsa-miR-148b-3p miRNAs, and a miRNA signature b) comprising hsa-miR-24-3p, hsa-miR-200b-3p and/or hsa-miR-99b-5p miRNAs. The authors have also found that determining the level of the miRNAs of the invention allows for the selection of a female subject as a candidate to receive a therapy to increase or restore endometrial receptivity.

Therefore, in a first aspect the invention relates to an *in vitro* method for determining endometrial receptivity in a female subject which comprises the steps of:
i) determining the level of the miRNAs from a miRNA signature, wherein said miRNA signature is selected from the group consisting of
   - a miRNA signature a) comprising the hsa-miR-24-3p, hsa-miR-200b-3p and/or hsa-miR-148b-3p miRNAs, and
   - a miRNA signature b) comprising the hsa-miR-24-3p, hsa-miR-200b-3p and/or hsa-miR-99b-5p miRNAs,
   in at least one sample from said subject, and
ii) comparing the level of said miRNAs obtained in step (i) with a reference value for each of the miRNAs,
   wherein a reduced level of hsa-miR-24-3p and/or hsa-miR-148b-3p, and/or an increased level of hsa-miR-200b-3p and/or hsa-miR-99b-5p, with respect to the reference value, is indicative that the endometrium is receptive, and
   wherein an increased level of hsa-miR-24-3p and/or hsa-miR-148b-3p, and/or a reduced level of hsa-miR-200b-3p and/or hsa-miR-99b-5p, with respect to the reference value, is indicative that the endometrium is not receptive.

In a second aspect, the present invention relates to an *in vitro* method for selecting a female subject as a candidate to receive a therapy to increase or restore endometrial receptivity which comprises
a. determining the endometrial receptivity of said subject by following the method according to the invention, and
b. selecting a subject as a candidate to receive an adequate therapy to increase or restore endometrial receptivity if the subject has no endometrial receptivity.

In another aspect, the invention relates to a kit comprising reagents adequate for the determination of the level of miRNAs forming part of a miRNA signature selected from the group consisting of a miRNA signature comprising the hsa-miR-24-3p, hsa-miR-200b-3p and hsa-miR-148b-3p miRNAs, and a miRNA signature comprising the hsa-miR-24-3p, hsa-miR-200b-3p and hsa-miR-99b-5p miRNAs.

In another aspect, the invention relates to the use of the kit of the invention for determining endometrial receptivity, or for identifying a woman as a candidate to receive a therapy to increase endometrial receptivity.

### BRIEF DESCRIPTION OF THE FIGURES

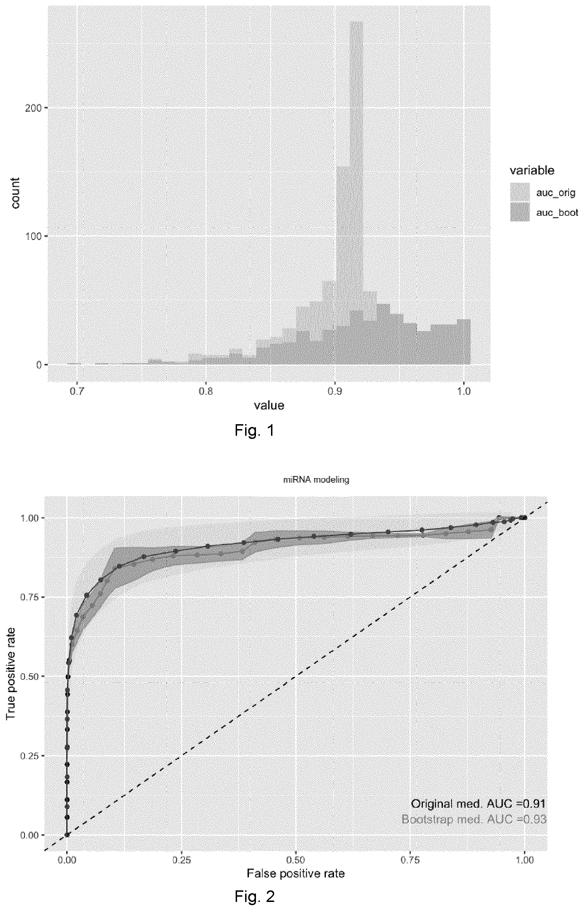

Figure 1. Graph representing a bootstrap simulation of up to 500 datasets with similar data distribution features and the given dataset for a miRNA signature consisting of miRNAs hsa-miR-24-3p, hsa-miR-200b-3p and hsa-miR-148b-3p obtained by method PBP-M. The original-data AUC tends to converge at 80% while the AUC of the simulated data returns a more normal distribution with a median AUC -80-90%. It shows that the model based in a miRNA signature consisting of miRNAs hsa-miR-24-3p, hsa-miR-200b-3p and hsa-miR-148b-3p is robust against data that fits within the given characteristics.
Figure 2. ROC curve for a bootstrap simulation of a miRNA signature consisting of miRNAs hsa-miR-24-3p, hsa-miR-200b-3p and hsa-miR-148b-3p obtained by method PBP-M. AUC values for the 500 replication bootstrap approaches are spread over the 0.8-1 range. It shows that the model based on a miRNA signature consisting of miRNAs hsa-miR-24-3p, hsa-miR-200b-3p and hsa-miR-148b-3p is significantly predictive (p-value 0.003259) with its reproducibility in the low-to-mid range (R2 0.43, adjusted R2 0.36).

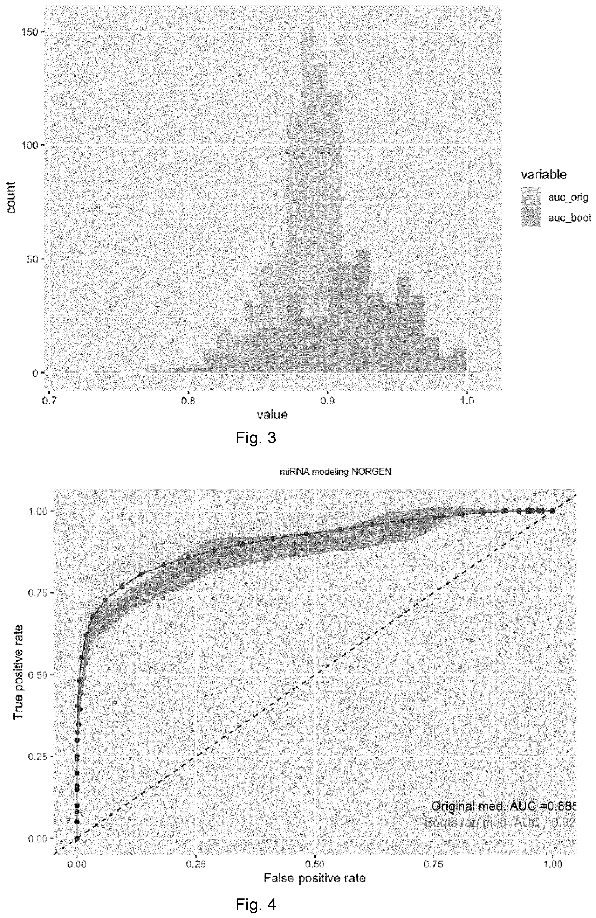

Figure 3. Graph representing a bootstrap simulation of up to 500 datasets with similar data distribution features and the given dataset for the miRNA signature consisting of miRNAs hsa-miR-24-3p, hsa-miR-200b-3p and hsa-miR-99b-5p obtained by method PBP-N. The original-data AUC tends to converge at 80% while the AUC of the simulated data returns a more normal distribution with a median AUC -80-90%. It shows that the model based in a miRNA signature consisting of miRNAs hsa-miR-24-3p, hsa-miR-200b-3p y hsa-miR-99b-5p is robust against data that fits within the given characteristics.
**Figure 4****.** ROC curve for bootstrap simulation of a miRNA signature consisting of miRNAs up to 500 datasets miRNAs hsa-miR-24-3p, hsa-miR-200b-3p y hsa-miR-99b-5p. AUC values for the 500 replication bootstrap approaches are spread over the 0.8-1 range. It shows that the model based on a miRNA signature consisting of miRNAs hsa-miR-24-3p, hsa-miR-200b-3p y hsa-miR-99b-5p is significantly predictive (p-value 0.00023) with its reproducibility in the low-to-mid range (R2 0.4935, adjusted R2 0.4392)

### DETAILED DESCRIPTION OF THE INVENTION

The authors of the present invention have found that a different stage of endometrial development can be elucidated by determination of the level of miRNAs. Accordingly, the present invention provides a method for determination of endometrial receptivity for embryo implantation and, thus, a method for prognosis of embryo implantation outcome and pregnancy. In other words, the levels of miRNAs of the invention can be associated with the status of the endometrium of a female subject, therefore, allowing a clinical decision if implantation-capable embryos are available for transfer into the uterus of the female subject. Thus, in the case of poor embryo implantation prognosis, the clinician may decide to cancel the embryo transfer, so that oocytes or embryos can be frozen and kept alive for the next transfer opportunity. On the other hand, in the case of good embryo implantation prognosis, the number of embryos which are to be transferred may be reduced. In particular, this method finds application in assisted reproduction centers and *in vitro* fertilization units for improving embryo implantation rates and pregnancy outcome.

The authors of the present invention have found that the receptivity status of an endometrium can be reliably assessed by determining the level of miRNAs from a miRNA signature which is selected from the group consisting of miRNA signature a) consisting of hsa-miR-24-3p, hsa-miR-200b-3p and/or hsa-miR-148b-3p miRNAs, and miRNA signature b) consisting of hsa-miR-24-3p, hsa-miR-200b-3p and/or hsa-miR-99b-5p miRNAs.

### 1. In vitro method for determining endometrial receptivity in a female subject

The term "endometrial receptivity", as used herein, relates to the state in which a woman's endometrium is prepared for embryo implantation. The term "endometrial receptivity" may be understood as a status during a period of time in which the endometrium acquires a functional status that allows blastocyst adhesion and leads to pregnancy. The term "endometrial receptivity" may also be understood as the stage of an endometrium at which an embryo transfer may lead to pregnancy irrespective of the cycle of *in vitro* fertilization (IVF) at which the implantation-capable embryo is transferred. Accordingly, the term "endometrial receptivity" refers to the status during a period of time in which the endometrium acquires a functional status that allows blastocyst adhesion and leads to a successful pregnancy in the same IVF cycle. Alternatively, the term "endometrial receptivity" refers to the status during a period of time in which the endometrium acquires a functional status that allows blastocyst adhesion and leads to pregnancy in a different IVF cycle, for example, by collecting a sample in a given IVF cycle and performing embryo transfer in the following IVF cycle.

The terms "microRNA" or "miRNA" or "miRNAs" or "miR", used interchangeably herein, are endogenous RNAs, some of which are known to regulate the expression of protein-coding genes at the post-transcriptional level. As used herein, the term "miRNA" refers to any type of micro-interfering RNA, including but not limited to, endogenous microRNA and artificial microRNA. Typically, endogenous miRNAs are small RNAs encoded in the genome which are capable of modulating the productive utilization of mRNA. A mature miRNA is a single-stranded RNA molecule of about 21-23 nucleotides in length which is complementary to a target sequence, and hybridizes to the target RNA sequence to inhibit its translation. miRNAs themselves are encoded by genes that are transcribed from DNA but not translated into protein (non-coding RNA); instead they are processed from primary transcripts known as pri-miRNA to short stem-loop structures called pre-miRNA and finally to functional miRNA. Mature miRNA molecules are partially complementary to one or more messenger RNA (mRNA) molecules, and their main function is to down-regulate gene expression. Besides their intracellular function, miRNAs can be exported or released by cells into the biological fluids, such as endometrial fluid, in very stable forms. The term "miRNA", as used herein, may refer to "cell-free miRNA", and relates to extracellular miRNA that circulates in biological fluids, such as endometrial fluid, in cell-free form or associated to extracellular vesicles.

Pre-miRNAs are referred to as "mir", whereas mature miRNAs are referred to as "miR". This prefix is followed by a dash and a number, the latter often indicating order of naming. miRNAs with nearly identical sequences except for one or two nucleotides are annotated with an additional lower case letter. Pre-miRNAs that lead to 100% identical mature miRNAs but that are located at different places in the genome are indicated with an additional dash-number suffix. Species of origin is designated with a three-letter prefix. Some "mir" can have two mature products. For example, hsa-mir-24-1 has two mature products, named hsa-miR-24-1-5p and hsa-miR-24-3p. The mature product annotated with 5p arises from the 5' arm of the mir-24-1 hairpin precursor, and the mature product annotated with 3p arises from the 3'arm of the mir-24-1 hairpin precursor. Nomenclature conventions used to describe miRNA sequences are described further in Ambros V et al., RNA, 2003, 9:277-279.

The term "hsa-miR-24-3p", as used herein, relates to the human miRNA as defined in the mIRBase database of the University of Manchester (http://www.mirbase.org/index.shtml) (release 21 as of April 2021) under accession number MIMAT0000080, as well as to homologs and orthologues thereof (>hsa-miR-24-3p; MIMAT0000080; UGGCUCAGUUCAGCAGGAACAG - SEQ ID NO:1).

The term "hsa-miR-200b-3p", as used herein, relates to the human miRNA as defined in the mIRBase database of the University of Manchester (http://www.mirbase.org/index.shtml) (release 21 as of April 2021) under accession number MIMAT0000318, as well as to homologs and orthologues thereof (>hsa-miR-200b-3p; MIMAT0000318; UAAUACUGCCUGGUAAUGAUGA - SEQ ID NO:2).

The term "hsa-miR-148b-3p", as used herein, relates to the human miRNA as defined in the mIRBase database of the University of Manchester (http://www.mirbase.org/index.shtml) (release 21 as of April 2021) under accession number MIMAT0000759, as well as to homologs and orthologues thereof (>hsa-miR-148b-3p; MIMAT0000759; UCAGUGCAUCACAGAACUUUGU - SEQ ID NO:3).

The term "hsa-miR-99b-5p", as used herein, relates to the human miRNA as defined in the mIRBase database of the University of Manchester (http://www.mirbase.org/index.shtml) (release 21 as of April 2021) under accession number MIMAT0000689, as well as to homologs and orthologues thereof (>hsa-miR-99b-5p; MIMAT0000689; CACCCGUAGAACCGACCUUGCG - SEQ ID NO:4).

In some embodiments, the term hsa-miR refers to miRNA in *Homo sapiens* as well as homologs and orthologues of the corresponding miRNA in other species of mammals.

As used herein, the term "microRNA signature" or "miRNA signature" refers to a "microRNA" or "miRNA" profile in a test sample relative to an appropriate control miRNA. In some embodiments, the miRNA signature is associated with a specific cellular or tissue condition which can be associated to a physiological or to a clinical condition. In some embodiments, the miRNA signature features or consists essentially of miRNAs that are individually or coordinately regulated and they are associated to a physiological or to a clinical condition. A miRNA expression signature may be used as a basis for computational methods or algorithms used for determination, prediction, prognosis, screening, early diagnosis, staging, therapy selection and monitoring of a status of an organ or a tissue or a cell type, for example, the uterus or the endometrium or endometrial cells. In one embodiment, the miRNA expression signature includes a profile of the level of one or more miRNA molecules. A "signature" is a group two or more genes or gene-related molecules (e.g., DNA, RNA, amino acids and proteins) that exist in a cell, tissue, fluid or other sample, characteristic of a particular cell or tissue or is characteristic of a particular physiological or disease status. The signature can be used to select or stratify a subject or group of subjects based on, for example, a physiological status, a specific stage of a disease, a risk of developing a particular disease or a probability or prediction of a prognosis, with sufficient accuracy to facilitate diagnosis or selection of treatment.

In one aspect, the miRNA signature is selected from the group consisting of a miRNA signature a) comprising the hsa-miR-24-3p, hsa-miR-200b-3p and/or hsa-miR-148b-3p miRNAs, and a miRNA signature b) comprising the hsa-miR-24-3p, hsa-miR-200b-3p and/or hsa-miR-99b-5p miRNAs.

The term "level" relates to the measurement of the amount of a nucleic acid, e.g. RNA or mRNA or miRNA, or of a protein. In the context of the invention, the level relates to the measurement of the amount of a nucleic acid, particularly RNA, more particularly miRNA, even more particularly hsa-miR-24-3p, hsa-miR-200b-3p, hsa-miR-148b-3p miRNAs or hsa-miR-99b-5p miRNAs.

According to the invention, measuring the level of the miRNA selected from the group consisting of miRNAs of miRNA signature a) and a miRNA signature b) of the invention in a biological sample obtained from the patient can be performed by a variety of techniques. According to standard methods, the nucleic acid contained in the samples (biological sample prepared from the patient) is first extracted, for example using lytic enzymes or chemical solutions or extracted by nucleic-acid-binding resins following the manufacturer's instructions. Conventional methods and reagents for isolating RNA from a biological sample are contemplated by the present invention and comprise without limitation mirVana^{™} PARIS^{™} Kit (ThermoFisher), Plasma/Serum RNA Purification Norgen kit (Norgenbiotek), High Pure miRNA Isolation Kit (Roche), Trizol (Invitrogen), Guanidinium thiocyanate-phenol-chloroform extraction, PureLink^{™} miRNA isolation kit (Invitrogen), PureLink Micro-to- Midi Total RNA Purification System (invitrogen), RNeasy kit (Qiagen), miRNeasy kit (Qiagen), Oligotex kit (Qiagen), phenol extraction, phenol-chloroform extraction, TCA/acetone precipitation, ethanol precipitation, Column purification, Silica gel membrane purification, Pure Yield^{™} RNA Midiprep (Promega), PolyATtract System 1000 (Promega), Maxwell(R) 16 System (Promega), SV Total RNA Isolation (Promega), geneMAG- RNA / DNA kit (Chemicell), TRI Reagent(R) (Ambion), RNAqueous Kit (Ambion), ToTALLY RNA^{™} Kit (Ambion), Poly(A)Purist^{™} Kit (Ambion) and any other methods, commercially available or not, known to the skilled person. The level of one or more miRNA in the biological sample may be determined by any suitable method. Any reliable method for measuring the level or amount of miRNA in a sample may be used. Generally, miRNA can be detected and quantified from a biological sample (including fractions thereof), such as samples of isolated by various methods known for mRNA, including, for example, amplification-based methods (e.g., Polymerase Chain Reaction (PCR), Real-Time Polymerase Chain Reaction (RT-PCR), Quantitative Polymerase Chain Reaction (qPCR), Real-Time Quantitative Polymerase Chain Reaction (RT-qPCR), rolling circle amplification, etc.), hybridization-based methods (e.g., hybridization arrays (e.g., microarrays), NanoString analysis, Northern Blot analysis, branched DNA (bDNA) signal amplification, in situ hybridization, etc.), and sequencing- based methods (e.g., next- generation sequencing methods, for example, using the Illumina or IonTorrent platforms). Other exemplary techniques include ribonuclease protection assay (RPA) and mass spectroscopy.

Many amplification-based methods exist for detecting the expression level of miRNA nucleic acid sequences, including, but not limited to, PCR, RT-PCR, qPCR, RT-qPCR and rolling circle amplification. Other amplification-based techniques include, for example, ligase chain reaction (LCR), multiplex ligatable probe amplification, in vitro transcription (IVT), strand displacement amplification (SDA), transcription-mediated amplification (TMA), nucleic acid sequence based amplification (NASBA), RNA (Eberwine) amplification, and other methods that are known to persons skilled in the art. A typical PCR reaction includes multiple steps, or cycles, that selectively amplify target nucleic acid species: a denaturing step, in which a target nucleic acid is denatured; an annealing step, in which a set of PCR primers (i.e., forward and reverse primers) anneal to complementary DNA strands, and an elongation step, in which a thermostable DNA polymerase elongates the primers. By repeating these steps multiple times, a DNA fragment is amplified to produce an amplicon, corresponding to the target sequence. Typical PCR reactions include 20 or more cycles of denaturation, annealing, and elongation. In many cases, the annealing and elongation steps can be performed concurrently, in which case the cycle contains only two steps. A reverse transcription reaction (which produces a cDNA sequence having complementarity to a miRNA) may be performed prior to PCR amplification. Reverse transcription reactions include the use of, e.g., a RNA-based DNA polymerase (reverse transcriptase) and a primer. Kits for quantitative real time PCR of miRNA are known, and are commercially available. Examples of suitable kits include, but are not limited to, the TaqMan(R) miRNA Assay (Applied Biosystems) and the mirVana^{™} qRT- PCR miRNA detection kit (Ambion). The miRNA can be ligated to a single stranded oligonucleotide containing universal primer sequences, a polyadenylated sequence, or adaptor sequence prior to reverse transcriptase and amplified using a primer complementary to the universal primer sequence, poly(T) primer, or primer comprising a sequence that is complementary to the adaptor sequence. In some embodiments, custom qRT-PCR assays can be developed for determination of miRNA levels. Custom qRT-PCR assays to measure miRNAs in a sample can be developed using, for example, methods that involve an extended reverse transcription primer and locked nucleic acid modified PCR. Custom miRNA assays can be tested by running the assay on a dilution series of chemically synthesized miRNA corresponding to the target sequence. This permits determination of the limit of detection and linear range of quantitation of each assay. Furthermore, when used as a standard curve, these data permit an estimate of the absolute abundance of miRNAs measured in the samples. Amplification curves may optionally be checked to verify that Ct values are assessed in the linear range of each amplification plot. Typically, the linear range spans several orders of magnitude. For each candidate miRNA assayed, a chemically synthesized version of the miRNA can be obtained and analyzed in a dilution series to determine the limit of sensitivity of the assay, and the linear range of quantitation. Relative expression levels may be determined, for example, according to the 2(-DELTADELTA C(T)) Method, as described by Livak et al, Analysis of relative gene expression data using real-time quantitative PCR and the 2(-DELTADELTA C(T)) Method. Methods (2001) Dec;25(4):402-8.

The term "sample" or "biological sample" or "sample tissue" or "sample fluid", as used herein, refers to a small part of a subject, representative of the whole and may be constituted by a biopsy or a body fluid sample. Biopsies are small pieces of tissue and may be fresh, frozen or fixed, such as formalin-fixed and paraffin embedded (FFPE). Body fluid samples may be blood, plasma, serum, urine, sputum, cerebrospinal fluid, milk, ductal fluid, uterine fluid, endometrial fluid samples and may likewise be fresh, frozen or fixed. Samples may be removed surgically, by extraction i.e. by hypodermic or other types of needles, by microdissection or laser capture. A body fluid may be obtained by non-surgical methods. Such is the case of endometrial fluid which can be obtained non-invasively by aspiration. The sample should contain any biological material suitable for detecting the desired biomarker (miRNA), thus, said sample should, advantageously comprise cell material from the subject. In some embodiments, the biological sample include endometrial tissue biopsy and/or endometrial fluid. Said sample is obtained for the purpose of the *in vitro* evaluation. In a preferred embodiment, the samples are obtained during the natural cycle, 16-21 days after the beginning of the menstruation. In some embodiments, endometrial fluid can be obtained any time during stimulated cycles and before embryo transfer. Methods for collecting and preparing these biological samples are known in the art.

The term "sample", as used herein, refers to biological material isolated from a subject. The sample can comprise cell and/or non-cell material of the subject, preferably non-cell material. In the present invention, the sample comprises genetic material, e.g., DNA, genomic DNA (gDNA), complementary DNA (cDNA), RNA, heterogeneous nuclear RNA (hnRNA), mRNA, etc., from the subject under study. In a particular embodiment, the genetic material is RNA. In a preferred embodiment the RNA is miRNA. In a further preferred embodiment, the miRNA of the sample originates from endometrial tissue and/or endometrial fluid. Methods for obtaining endometrial samples are well known to those skilled in the art.

The term "subject" or "individual" or "animal" or "patient" or "mammal" is understood as any subject, particularly a mammalian subject, for whom determination or diagnosis or prognosis is desired. Mammalian subjects include humans, domestic animals, farm animals, and zoo, sports, or pet animals such as dogs, cats, guinea pigs, rabbits, rats, mice, horses, cattle, cows, and so on. In a preferred embodiment of the invention, the subject is a mammal. In a more preferred embodiment of the invention, the subject is a human.

As used herein the term "female subject" refers to a mammalian female whose endometrial receptivity is to be determined. Typically said mammal is a human (i.e. a woman), but may concern other mammals such as primates, dogs, cats, pigs, sheep, cows.

The term "reference value" or "reference level", as used herein in the context of the methods of the invention, relates to a value used as a reference for the level values/data obtained from samples of subjects to be classified within a certain physiological or clinical status or to be classified as having high or low probability of entering a physiological or clinical status, or to be classified as having good or bad prognosis for entering a physiological or clinical status. The reference value or reference level according to the any of the methods of the invention can be an absolute value; a relative value; a value that has an upper and/or lower limit; a range of values; an average value; a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from a population of subjects of the chronological age matched group, or based on a pool of samples including or excluding the sample to be tested. Various considerations are taken into account when determining the reference value of the marker. Among such considerations are the age, weight, sex, general physical condition of the patient and the like. For example, equal amounts of a group of at least 2, at least 10, at least 100 to preferably more than 1000 subjects, preferably classified according to the foregoing considerations, for example according to various age categories, are taken as the reference group.

The term "reference value" as used herein, refers to the level of the miRNA under consideration in a reference sample. A "reference sample", as used herein, means a sample obtained from subjects, preferably two or more subjects, known to be free of disease or, alternatively, to have a known physiological or clinical status. The suitable reference expression levels of miRNAs can be determined by measuring the expression levels of said miRNAs in several suitable subjects, and such reference levels can be adjusted to specific subject populations. In a preferred embodiment, the reference sample is obtained from a patient undergoing assisted reproduction procedures.

A reference value is determined for each miRNA. Typically, the reference value can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skill in the art. The threshold value has to be determined in order to obtain the optimal sensitivity and specificity according to the function of the test and the benefit/risk balance (clinical consequences of false positive and false negative). Typically, the optimal sensitivity and specificity (and so the threshold value) can be determined using a Receiver Operating Characteristic (ROC) curve based on experimental data. Preferably, the person skilled in the art may compare the miRNAs levels (obtained according to the method of the invention with a defined threshold value). In one embodiment of the present invention, the threshold value is derived from the miRNA level (or ratio, or score) determined in a biological sample derived from one or more patients with a high potential for pregnancy outcome. In one embodiment of the present invention, the threshold value may also be derived from the miRNA level (or ratio, or score) determined in a biological sample derived from one or more patients with a low potential for pregnancy outcome. Furthermore, retrospective measurement of the miRNA levels (or ratio, or scores) in properly banked historical patient samples may be used in establishing these threshold values.

In some embodiments, the reference value may be determined by carrying out a method comprising the steps of a) providing a collection of biological samples obtained from patients during the implantation window, b) providing, for each sample provided at step a), information relating to the actual clinical outcome (pregnancy or no pregnancy); c) providing a series of arbitrary quantification values; d) determining the level of the miRNA for each sample contained in the collection provided at step a); e) classifying said samples in two groups for one specific arbitrary quantification value provided at step c), respectively: (i) a first group comprising samples that exhibit a quantification value for the level that is lower than the said arbitrary quantification value contained in the said series of quantification values; (ii) a second group comprising samples that exhibit a quantification value for said level that is higher than the said arbitrary quantification value contained in the said series of quantification values; whereby two groups of samples are obtained for the said specific quantification value, wherein the samples of each group are separately enumerated; f) calculating the statistical significance between (i) the quantification value obtained at step e) and (ii) the actual clinical outcome of the patients (i.e. pregnancy or no pregnancy) from which samples contained in the first and second groups defined at step f) derive; g) reiterating steps f) and g) until every arbitrary quantification value provided at step d) is tested; and h) setting the said reference value as consisting of the arbitrary quantification value for which the highest statistical significance (most significant) has been calculated at step g).

Alternatively, it is possible that the reference sample is obtained by pooling samples from normal or healthy subjects or by pooling samples from patients which are undergoing assisted reproduction procedures and/or IVF. The expression profile of the miRNAs in the reference sample can, preferably, be generated from a population of two or more subjects; for example, the population can comprise 3, 4, 5, 10, 15, 20, 30, 40, 50 or more subjects. The setting of a single "cut-off value" thus allows discrimination between a high potential for pregnancy outcome (receptive endometrium) or low potential for pregnancy outcome (non-receptive endometrium).

In some embodiments, the reference value may correspond to the level of miRNA determined in a biological sample associated with a high or a low potential for pregnancy outcome. In some embodiments, the reference value is the level of the miRNA determined in a sample of endometrial fluid from a receptive endometrium, preferably, from a receptive endometrium from a subject who achieved pregnancy in the same cycle in which the reference sample was obtained.

In some embodiments, a score which is a composite of the expression levels of the different miRNAs may also be determined and compared to a reference value wherein a difference between said score and said reference value is indicative of a receptive endometrium (or a high potential for pregnancy outcome of the subject) or is indicative of a non-receptive endometrium (or with a low potential for pregnancy outcome of the subject). In some embodiments, the method of the invention comprises the step of determining the patient pregnancy outcome using a classification algorithm.

The term "level" relates to the measurement of the amount of a nucleic acid, e.g. RNA or mRNA, or of a protein. In the context of the invention, the term "level" or "level of the miRNAs" relates to the measurement of the amount of a nucleic acid, particularly RNA, more particularly miRNA, even more particularly hsa-miR-24-3p, hsa-miR-200b-3p, hsa-miR-148b-3p and/or hsa-miR-99b-5p miRNAs.

The term "reduced level" or "reduced level of miRNA" as used herein in relation to the level of a miRNA, particularly hsa-miR-24-3p, hsa-miR-200b-3p, hsa-miR-148b-3p and/or hsa-miR-99b-5p, relates to a situation where the level of expression of said miRNA is decreased at least 5%, at least 10%, at least 20%, at least 30%, at least 40%, at least 50%, at least 60%, at least 70%, at least 80%, at least 90% or at least 100% when compared to the corresponding reference value.

The term "increased level" or "increased level of miRNA" as used herein in relation to the level of a miRNA, particularly hsa-miR-24-3p, hsa-miR-200b-3p, hsa-miR-148b-3p and/or hsa-miR-99b-5p, relates to a situation where the level of expression of said miRNA is increased at least 5%, at least 10%, at least 20%, at least 30%, at least 40%, at least 50%, at least 60%, at least 70%, at least 80%, at least 90% or at least 100% when compared to the corresponding reference value.

The term "endometrium is receptive" as used herein in relation to the level of one or more miRNAs, particularly hsa-miR-24-3p, hsa-miR-200b-3p, hsa-miR-148b-3p and/or hsa-miR-99b-5p, relates to a situation where the level of hsa-miR-24-3p and/or hsa-miR-148b-3p is reduced at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-200b-3p is increased at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-24-3p is reduced at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-200b-3p and/or hsa-miR-99b-5p is increased at least 5%, at least 10%, at least 20%, at least 30%, at least 40%, at least 50%, at least 60%, at least 70%, at least 80%, at least 90% or at least 100% when compared to the corresponding reference value.

The term "endometrium is not receptive" as used herein in relation to the level of a miRNA, particularly hsa-miR-24-3p, hsa-miR-200b-3p, hsa-miR-148b-3p and/or hsa-miR-99b-5p, relates to a situation where the level of hsa-miR-24-3p and/or hsa-miR-148b-3p is increased at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-200b-3p is reduced at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-24-3p is increased at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-200b-3p and/or hsa-miR-99b-5p is reduced at least 5%, at least 10%, at least 20%, at least 30%, at least 40%, at least 50%, at least 60%, at least 70%, at least 80%, at least 90% or at least 100% when compared to the corresponding reference value.

The expression "determining the endometrial receptivity" as used herein relates to the assessment or prediction of a physiological or clinical status of the endometrium, in particular, with regards to the pregnancy outcome (e.g. likelihood of pregnancy or implantation-capable embryo survival).

The term "endometrium is receptive" as used herein relates to the situation in which the endometrium of the female subject shows at least 5%, at least 10%, at least 20%, at least 30%, at least 40%, at least 50%, at least 60%, at least 70%, at least 80%, at least 90%, at least 100% probabilities of acquiring a functional status that, upon transfer of a implantation-capable embryo, allows blastocyst adhesion and that will lead to pregnancy.

The term "endometrium is not receptive" as used herein relates to the situation in which the endometrium of the female subject shows at least 5%, at least 10%, at least 20%, at least 30%, at least 40%, at least 50%, at least 60%, at least 70%, at least 80%, at least 90%, at least 100% probabilities of not acquiring a functional status that, upon transfer of a implantation-capable embryo, allows blastocyst adhesion and that will lead to pregnancy. The term "pregnancy" is understood as the visualization of a gestational sac 4 weeks after embryo transfer.

As the person skilled in the art will understand, such determination does not usually seek to be correct for all (i.e., 100%) of the subjects that are going to be identified. However, the term requires that a statistically significant part of the subjects can be identified (for example, a cohort in a cohort study). The person skilled in the art can easily determine if a part is statistically significant using several well-known statistical evaluation tools, for example, determination of confidence intervals, determination of p values, Student's t-test, Mann-Whitney test, etc. The details are found in Dowdy and Wearden, Statistics for Research, John Wiley and Sons, New York 1983. The preferred confidence intervals are at least 90%, at least 95%, at least 97%, at least 98% or at least 99%. The p values are preferably 0.1, 0.05, 0.01, 0.005 or 0.0001. More preferably, at least 60%, at least 70%, at least 80% or at least 90% of the subjects of a population can be suitably identified by the method of the present invention.

The method of the present invention is particularly suitable for reaching a clinical decision. As used herein the term "clinical decision" refers to any decision to take or not take an action that has an outcome that affects the health or survival of the embryo. In particular, in the context of the invention, a clinical decision refers to a decision to transfer or not the embryo in the uterus of a female subject. In particular, the method of the present invention will thus help to avoid the transfer into the uterus of embryos with a low potential for pregnancy outcome. The method of the present invention is also particularly suitable for enhancing pregnancy outcome. The method of the present invention is also particularly suitable for preventing or reducing the implantation failure. The method of the present invention is also particularly suitable for improving in vitro fertilization outcomes.

Thus, in a first aspect, the invention relates to an *in vitro* method for determining endometrial receptivity in a female subject (first method of the invention), which comprises the steps of:
(i) determining the level of the miRNAs from a miRNA signature, wherein said miRNA signature is selected from the group consisting of
   a miRNA signature a) comprising the hsa-miR-24-3p, hsa-miR-200b-3p and/or hsa-miR-148b-3p miRNAs, and
   a miRNA signature b) comprising the hsa-miR-24-3p, hsa-miR-200b-3p and/or hsa-miR-99b-5p miRNAs,
   in at least one sample from said subject, and
(ii) comparing the level of said miRNAs obtained in step (i) with a reference value for each of the miRNAs,
   wherein a reduced level of hsa-miR-24-3p and/or hsa-miR-148b-3p, and/or an increased level of hsa-miR-200b-3p and/or hsa-miR-99b-5p, with respect to the reference value, is indicative that the endometrium is receptive, and
   wherein an increased level of hsa-miR-24-3p and/or hsa-miR-148b-3p, and/or a reduced level of hsa-miR-200b-3p and/or hsa-miR-99b-5p, with respect to the reference value, is indicative that the endometrium is not receptive.

In a particular embodiment, signature a) comprises or consists of the hsa-miR-24-3p or the hsa-miR-200b-3p or the hsa-miR-148b-3p miRNAs. In a more particular embodiment, signature a) comprises or consists of the hsa-miR-24-3p and the hsa-miR-200b-3p; or the hsa-miR-24-3p and the hsa-miR-148b-3p miRNAs; or the hsa-miR-200b-3p and hsa-miR-148b-3p miRNAs. In an even more particular embodiment, signature a) comprises or consists of the hsa-miR-24-3p, hsa-miR-200b-3p and hsa-miR-148b-3p miRNAs.

In a more particular embodiment, signature a) comprises or consists of the hsa-miR-24-3p and the hsa-miR-200b-3p; or the hsa-miR-24-3p and the hsa-miR-148b-3p miRNAs; or the hsa-miR-200b-3p and hsa-miR-148b-3p miRNAs. In an even more particular embodiment, signature a) comprises or consists of the hsa-miR-24-3p, hsa-miR-200b-3p and hsa-miR-148b-3p miRNAs.

In a particular embodiment, signature b) comprises or consists of the hsa-miR-24-3p or hsa-miR-200b-3p or hsa-miR-99b-5p miRNAs. In a more particular embodiment, signature b) comprises or consists of the has-miR-24-3p and the hsa-miR-200b-3p; or the hsa-miR-24-3p and the hsa-miR-99b-5p miRNAs; or the hsa-miR-200b-3p and hsa-miR-99b-5p miRNAs. In an even more particular embodiment, signature b) comprises or consists of the hsa-miR-24-3p, hsa-miR-200b-3p and hsa-miR-99b-5p miRNAs.

In a particular embodiment, a reduced level of the hsa-miR-24-3p miRNA with respect to the reference value is indicative that the endometrium is receptive, and an increased level of the hsa-miR-24-3p miRNA with respect to the reference value is indicative that the endometrium is not receptive.

In a particular embodiment, a reduced level of the hsa-miR-148b-3p with respect to the reference value is indicative that the endometrium is receptive, and an increased level of the hsa-miR-148b-3p miRNA with respect to the reference value is indicative that the endometrium is not receptive.

In a particular embodiment, an increased level of the hsa-miR-200b-3p with respect to the reference value is indicative that the endometrium is receptive, and a reduced level of the hsa-miR-200b-3p miRNA with respect to the reference value is indicative that the endometrium is not receptive.

In a particular embodiment, an increased level of the hsa-miR-99b-5p with respect to the reference value is indicative that the endometrium is receptive, and a reduced level of the hsa-miR-99b-5p miRNA with respect to the reference value is indicative that the endometrium is not receptive.

In a particular embodiment, a reduced level of the hsa-miR-24-3p and an increased level of the hsa-miR-200b-3p is indicative that the endometrium is receptive, and an increased level of the hsa-miR-24-3p and a reduced level of the hsa-miR-200b-3p is indicative that the endometrium is not receptive.

In a particular embodiment, a reduced level of the hsa-miR-24-3p and the hsa-miR-148b-3p is indicative that the endometrium is receptive, and an increased level of the hsa-miR-24-3p and the hsa-miR-148b-3p is indicative that the endometrium is not receptive.

In a particular embodiment, an increased level of the hsa-miR-200b-3p and a reduced level of hsa-miR-148b-3p is indicative that the endometrium is receptive, and a reduced level of the hsa-miR-200b-3p and an increased level of the hsa-miR-148b-3p is indicative that the endometrium is not receptive.

In a particular embodiment, a reduced level of the hsa-miR-24-3p and an increased level of hsa-miR-99b-5p is indicative that the endometrium is receptive, and an increased level of the hsa-miR-24-3p and a reduced level of the hsa-miR-99b-5p is indicative that the endometrium is not receptive.

In a particular embodiment, an increased level of hsa-miR-200b-3p and hsa-miR-99b-5p is indicative that the endometrium is receptive, and a reduced level of hsa-miR-200b-3p hsa-miR-99b-5p is indicative that the endometrium is not receptive.

In a particular embodiment, a reduced level of the hsa-miR-24-3p and hsa-miR-148b-3p and an increased level of the hsa-miR-200b-3p is indicative that the endometrium is receptive, and an increased level of the hsa-miR-24-3p and hsa-miR-148b-3p and a reduced level of the hsa-miR-200b-3p is indicative that the endometrium is not receptive.

In a particular embodiment, a reduced level of hsa-miR-24-3p and an increased level of hsa-miR-200b-3p and hsa-miR-99b-5p miRNAs is indicative that the endometrium is receptive and, an increased level of hsa-miR-24-3p and a reduced level of hsa-miR-200b-3p and hsa-miR-99b-5p miRNAs is indicative that the endometrium is not receptive.

In a first step of the first method of the invention for determining endometrial receptivity in a female subject, the expression level of miRNAs is determined in a sample from a subject whose endometrial receptivity is to be determined.

Before analyzing the sample, it will often be desirable to perform one or more preparation operations upon said sample aimed at separating the molecule to be determined (RNA, in particular miRNA) from other molecules found in the sample.

Typically, these sample preparation operations include manipulations such as concentration, suspension, extraction of intracellular material, e.g., nucleic acids from tissue/whole cell/fluid samples and the like, amplification of nucleic acids, fragmentation, transcription, labeling and/or extension reactions. Nucleic acids, especially RNA can be isolated using any techniques known in the art. There are two main methods for isolating RNA: (i) phenol-based extraction and (ii) silica matrix or glass fiber filter (GFF)-based binding. Phenol-based reagents contain a combination of denaturants and RNase inhibitors for cell and tissue disruption and subsequent separation of RNA from contaminants. Phenol-based isolation procedures can recover RNA species in the 10-200-nucleotide range e.g., miRNAs, 5S rRNA, 5.8S rRNA, and U1 snRNA. If a sample of "total" RNA was purified by the popular silica matrix column or GFF procedure, it may be depleted in small RNAs. Extraction procedures such as those using Trizol or TriReagent, however will purify all RNAs, large and small, and are the recommended methods for isolating total RNA from biological samples that will contain miRNAs/siRNAs. These methods are typically well known by a person skilled in the art. There are also commercial kits available for miRNA purification including, without limitation, miRNeasy Mini kit from Qiagen, miRNA isolation kits from Life Technologies, mirPremier microRNA isolation kit from Sigma-Aldrich and High Pure miRNA isolation kit from Roche.

In one embodiment, miRNA isolation is performed by method based on the organic extraction with acid phenol-chloroform and beta-mercaptoethanol), such as for example mirVana^{™} PARIS^{™} Kit (ThermoFisher). In another embodiment, miRNA isolation is performed by method based on the use of a column chromatography that uses silicon carbide, such as the Plasma/Serum RNA Purification Norgen kit (Norgenbiotek). In another embodiment, miRNA isolation is performed by both methods mirVana^{™} PARIS^{™} Kit (ThermoFisher) and Plasma/Serum RNA Purification Norgen kit (Norgenbiotek).

In one embodiment, determination of the level of miRNA hsa-miR-99b-5p requires RNA extraction from a sample of the female subject, wherein the RNA extraction from the sample does not comprise the use of acid phenol-chloroform and β-mercaptoethanol (also 2-Mercaptoethanol). In a more particular embodiment, the RNA extraction is made by a method based on a silicon carbide column chromatography.

Determination of the levels of RNA, in particular the levels of miRNA, can be carried out by any method known in the art such as RT-qPCR, northern blot, RNA dot blot, TaqMan, tag based methods such as serial analysis of gene expression (SAGE) including variants such as LongSAGE and SuperSAGE, microarrays. Determination of the mRNA levels can also be carried out by fluorescence in situ hybridization (FISH). The detection can be carried out in individual samples or in tissue microarrays. In a particular embodiment, the levels of miRNAs in a sample from a subject in which the endometrial receptivity is to be determined by real-time PCR (RT-PCR). In another particular embodiment, the levels of the miRNAs are determined by miRNA microarray.

In a particular embodiment, the methods of the invention comprise the steps of providing total RNAs extracted from an endometrial tissue or endometrial fluid samples and subjecting the RNAs to amplification and hybridization to specific probes, more particularly by means of a quantitative or semi-quantitative real time polymerase chain reaction (RT-PCR).

The levels of miRNA gene transcripts can be quantified in comparison with an internal standard, for example, the level of at least one mRNA from a "housekeeping" gene or of at least one miRNA in the same sample. A suitable "housekeeping" gene for use as an internal standard includes, e.g., myosin or glyceraldehyde-3-phosphate dehydrogenase (GAPDH). The methods for quantitative RT-PCR and variations thereof are within the skill in the art. The three commonly used methods of quantitative polymerase chain reaction are through agarose gel electrophoresis, the use of SYBR Green (a double stranded DNA dye), and the fluorescent reporter probe. The latter two of these three can be analyzed in real-time, constituting real-time polymerase chain reaction method. Using SYBR Green dye gives results in real time. A preferred embodiment of the present invention regards the real-time quantitative RT-PCR method, based on the use of either SYBR Green dye or a dual-labelled probe for the detection and quantification of nucleic acids.

In the method of the invention, cells, fluid or other biological samples as described above can further comprise suitable controls. Such suitable controls will be obvious to one skilled in the art and are considered part of the common knowledge. The relative miRNA level in the control or normal samples can further be determined with respect to one or more RNA expression standards. The standards can comprise, for example, a zero miRNA level, the miRNA level in a standard cell line, or the average level of miRNA previously obtained for a population of normal human controls.

In order to normalize the values of level of miRNA among the different samples, it is possible to compare the levels of the miRNA of interest in the test samples with the level of a control RNA or reference RNA. Thus, in the context of the invention, the term "normalized" level refers to the level of at least one additional miRNA relative to the level of a single reference or control RNA, or a particular set of reference or control RNAs.

A "control RNA" or a "reference RNA", as used herein, relates to RNA whose level does not change or changes only in limited amounts in samples under analysis with respect to control samples. In the analysis of miRNAs, various strategies can be used for data normalization, ranging from the use of particular endogenous miRNA or small RNA controls, external spike-in molecules or measures of central tendency. When quantifying *cellular* miRNAs, stable small RNA controls are currently used as reference RNAs. These include small noncoding RNAs, and specifically small nuclear RNA and small nucleolar RNA such as SNORD44 (RNU44), SNORD48 (RNU48) and RNU6-1 (Mamm U6). The most common choice could be the use of a miRNA that does not vary considerably between individuals.

In a preferred embodiment, normalization is carried out using the levels of hsa-miR-200c-3p, of hsa-miR-92a-3p or of a combination thereof.

In one embodiment, relative miRNA level quantification is calculated according to the comparative threshold cycle (Ct) method using miRNAs as controls. Final results are determined according to the formula 2-ΔCt, where ΔCT values of the sample are determined by subtracting the Ct value of the target gene from the value of the control miRNA.

In a second step of the first method of the invention for determining endometrial receptivity in a female subject, the level of expression of miRNAs in a sample from a subject in which the receptivity of the endometrium is to be determined is compared to a reference value, wherein a reduced level of hsa-miR-24-3p and/or hsa-miR-148b-3p, and/or an increased level of hsa-miR-200b-3p, and/or a reduced level of hsa-miR-24-3p, and/or an increased level of hsa-miR-200b-3p and/or hsa-miR-99b-5p, with respect to the reference value, is indicative that the endometrium is receptive, and wherein an increased level of hsa-miR-24-3p and/or hsa-miR-148b-3p, and/or a reduced level of hsa-miR-200b-3p, and/or an increased level of hsa-miR-24-3p, and/or a reduced level of hsa-miR-200b-3p and/or hsa-miR-99b-5p, with respect to the reference value, is indicative that the endometrium is not receptive.

In a particular embodiment of the first method of the invention, the reference value is the level of the miRNAs hsa-miR-24-3p, hsa-miR-200b-3p, hsa-miR-148b-3p and/or hsa-miR-99b-5p determined in a sample from a subject or a pool of subjects in which the endometrium is receptive.

In an alternative embodiment, the reference value is the level of the miRNAs hsa-miR-24-3p, hsa-miR-200b-3p, hsa-miR-148b-3p and/or hsa-miR-99b-5p determined in a sample from a subject or a pool of subjects in which the endometrium is not receptive. According to this alternative embodiment, the term "endometrium is receptive" as used herein in relation to the level of one or more miRNAs, particularly hsa-miR-24-3p, hsa-miR-200b-3p, hsa-miR-148b-3p and/or hsa-miR-99b-5p, relates to a situation where the level of hsa-miR-24-3p and/or hsa-miR-148b-3p is increased at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-200b-3p is reduced at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-24-3p is increased at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-200b-3p and/or hsa-miR-99b-5p is reduced at least 5%, at least 10%, at least 20%, at least 30%, at least 40%, at least 50%, at least 60%, at least 70%, at least 80%, at least 90% or at least 100% when compared to the corresponding reference value. According to this alternative embodiment, the term "endometrium is not receptive" as used herein in relation to the level of a miRNA, particularly hsa-miR-24-3p, hsa-miR-200b-3p, hsa-miR-148b-3p and/or hsa-miR-99b-5p, relates to a situation where the level of hsa-miR-24-3p and/or hsa-miR-148b-3p is reduced at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-200b-3p is increased at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-24-3p is reduced at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-200b-3p and/or hsa-miR-99b-5p is increased at least 5%, at least 10%, at least 20%, at least 30%, at least 40%, at least 50%, at least 60%, at least 70%, at least 80%, at least 90% or at least 100% when compared to the corresponding reference value.

Said reference value sample is typically obtained by combining equal amounts of samples from a subject population. In a particular preferred embodiment, the reference value is the level of expression of hsa-miR-24-3p, hsa-miR-200b-3p and/or hsa-miR-148b-3p and/or hsa-miR-99b-5p determined in a sample from one or more subjects in which the endometrium is receptive and transfer of an implantation-capable embryo results in pregnancy.

Once the reference value of the levels of hsa-miR-24-3p, hsa-miR-200b-3p, hsa-miR-148b-3p and/or hsa-miR-99b-5p is established, the levels of hsa-miR-24-3p, hsa-miR-200b-3p, hsa-miR-148b-3p and/or hsa-miR-99b-5p in a sample from a subject in which the receptivity of the endometrium is to be determined can be compared with this reference value, and thus be assigned as "increased", "decreased" or "equal". For example, an increase in levels above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" level. On the other hand, a decrease in levels below the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" level. Levels can be seen as "equal" to the reference value if the levels differ with respect to the reference value less than 5%, less than 4%, less than 3%, less than 2%, less than 1%, less than 0.5 %, less than 0.4%, less than 0.3%, less than 0.1%, less than 0.05%, or less.

In another embodiment, the female subject is undergoing an in vitro fertilization procedure.

In vitro fertilization (IVF) is the joining of a woman's egg and a man's sperm in a laboratory dish. In vitro fertilization (IVF) is a form of assisted reproductive technology (ART). IVF involves special medical techniques which are used to help a woman become pregnant. There are five basic steps to an IVF cycle: Step 1: Stimulation, also called super ovulation; Step 2: Egg retrieval; Step 3: Insemination and Fertilization; Step 4: Embryo culture; Step 5: Embryo transfer.

In a preferred embodiment, the levels of the miRNAs are indicative of receptivity within the same *in vitro* fertilization cycle in which the sample from the female subject has been obtained.

In a preferred embodiment, the sample is endometrial fluid.

The term "endometrial fluid" or "endometrial cavity fluid (ECF)" or "uterine fluid" or ""uterine cavity fluid" or "uterine secretion" or "endometrial aspirate" refers to the fluid accumulation within the endometrial cavity which can be collected in a non-invasive manner, for example, by aspiration, but also by uterine flushing and/or washing with physiological saline solution. In some embodiments, uterine cavity lavage samples or endometrial fluid with no lavage are samples in the method of the present invention. The term uterine fluid may refer to a protein-rich histotroph that contains secretions from the endometrial glands and cleavage products of both the secreted proteins and the glycocalyx (the glycoprotein mucin-rich layer coating the endometrial apical cell surface).

In most cases, the clinician will examine the inside lining of the uterus before start of an IVF cycle. This might involve a sonohysterography, in which fluid is injected through the cervix into the uterus, and an ultrasound to create images of the uterine cavity. Alternatively, the procedure may include a hysteroscopy, in which a thin, flexible, lighted telescope (hysteroscope) is inserted through the vagina and cervix into the uterus. However, this approach does not provide information with regards to the molecular profile of the endometrium. Nevertheless, most techniques used to specifically investigate endometrial receptivity require invasive procedures and collection of an endometrial biopsy. The biopsy collection procedure represents a hindrance to performing embryo transfer (ET) in the same IVF cycle at which the biopsy was collected. In other words, if endometrial receptivity is assessed by analysis of a biopsy sample, embryo transfer has to be delayed to the next IVF cycle.

The authors of the present invention have developed a non-invasive technique for analyzing endometrial miRNAs in endometrial fluid aspirate (EFA) obtained during the window of implantation, which is a non-invasive, non-surgical sample collection approach. Thus, the authors have developed a method to identify a receptive endometrium without the need of damaging the endometrial tissue by the biopsy collection procedure, which otherwise represents a hindrance for embryo implantation. Following the method of the invention, embryo transfer can be performed in the same cycle of IVF as the IVF cycle in which a sample of endometrial fluid is non-invasively collected. This approach is, in particular, advantageous because the period between the clinical decision to proceed with embryo transfer, and the transfer itself, can be substantially reduced. Considering that the conventional approach of biopsy analysis requires several weeks from analysis to embryo transfer, the present invention minimizes the risk of potential worsening of the endometrium by reducing the period between sample analysis and embryo transfer, potentially, down to only hours. The stage of the endometrium at which i) collection of a sample of endometrial fluid, ii) determination according to the first method of the invention of the levels of miRNAs in said sample of endometrial fluid and iii) embryo transfer of an implantation-capable embryo into the uterus of the subject, all performed in the same IVF cycle, may be understood according to the invention as the stage of receptive endometrium (also "*implantative* endometrium"). Notwithstanding, the present method also finds application in the cases in which the embryo transfer is performed in the following IVF cycle after the cycle in which the sample of endometrial fluid is collected.

In a further preferred embodiment, the sample of endometrial fluid comprises free and extracellular vesicle-associated miRNAs.

In another embodiment, prior to step i) according to the first method of the invention, the method comprises a non-invasive step of collecting at least one sample of endometrial fluid comprising free and extracellular vesicle-associated miRNAs.

The authors have found that the method of the invention can be applied to the analysis of the level of free and extracellular vesicle-associated miRNAs from the endometrial fluid of women so as to elaborate a prognosis whether the endometrium is functionally capable for embryo implantation or not. The method provides a non-invasive approach for improving embryo implantation rates to prevent couples undergoing in vitro fertilization (IVF) techniques from losing embryos when they are transferred to an endometrium that is not prepared for implantation. In this way, the method of the invention prevents the woman from undergoing hormone treatment and the surgical process to perform another IVF cycle and obtain more embryos.

The term extracellular vesicle (EV) includes a microvesicle (e.g. any vesicle shed from the plasma membrane of a cell), an exosome (e.g. any vesicle derived from the endo-lysosomal pathway), an apoptotic body (e.g. obtainable from apoptotic cells), a microparticle (which may be derived from e.g. platelets), an ectosome (derivable from e.g. neutrophils and monocytes in serum), prostatosome (e.g. obtainable from prostate cancer cells), a cardiosome (e.g. derivable from cardiac cells), microvesicles particles apoptotic bodies, argosomes, blebbing vesicles, budding vesicles, dexosomes, exosomes-like vesicles, exosomes, exovesicles, extracellular membrane vesicles, matrix vesicles, membrane particules, membrane vesicles, microparticules, microvesicles, nanovesicles, oncosomes, prominosomes, prostasomes, shedding microvesicles, shedding vesicles, and tolerosomes, etc. The term extracellular vesicle (EV) may relate to any type of lipid-based structure (with vesicular morphology or with any other type of suitable morphology) that can act as a delivery or transport vehicle for a miRNA of interest, in particular, any lipid-based structure containing a miRNA of interest and found in endometrial fluid.

In some embodiments, the mean of the EVs size is from 10 ± 0.1 nm to 1000 ± 0.1 nm, preferably from 50 ± 0.1 nm to 500 ± 0.1 nm, more preferably from 100 ± 0.1 nm to 400 ± 0.1 nm, yet more preferred from 150 ± 0.1 nm to 350 ± 0.1 nm.

"Free miRNA" or "cell-free miRNA" is understood as miRNA which is not associated to any defined cellular structure or organelle. In some embodiments, free miRNA refers to miRNA present in a biological fluid. In a preferred embodiment, free miRNA is miRNA present in endometrial fluid.

Isolation, purification, and enrichment can be done in a general and non-selective manner (typically including serial centrifugation). Alternatively, isolation, purification, and enrichment can be done in a more specific and selective manner (e.g., using producer cell-specific surface markers). For example, specific surface markers may be used in immunoprecipitation, FACS sorting, affinity purification, bead-bound ligands for magnetic separation etc. In some embodiments, size exclusion chromatography can be utilized to isolate or purify the EVs. Size exclusion chromatography techniques are known in the art. Exemplary, non-limiting techniques are provided herein. In some embodiments, a void volume fraction is isolated and comprises the EVs of interest. In some embodiments, for example, density gradient centrifugation can be utilized to further isolate the EVs. Still further, in some embodiments, it can be desirable to further separate the producer cell derived EVs, from EVs of other origin. For example, the producer cell-derived EVs, can be separated from non-producer cell-derived EVs, e.g., exosomes, by immunosorbent capture using an antigen antibody specific for the producer cell.

In some embodiments, the isolation of EVs may involve size exclusion chromatography or ion chromatography, such as anion exchange, cation exchange, or mixed mode chromatography. In some embodiments, the isolation of EVs may involve desalting, dialysis, tangential flow filtration, ultrafiltration, or diafiltration, or any combination thereof. In some embodiments, the isolation of EVs may involve combinations of methods that include, but are not limited to, differential centrifugation, size-based membrane filtration, concentration and/or rate zonal centrifugation. In some embodiments, the isolation of EVs may involve one or more centrifugation steps. The centrifugation may be performed at about 50,000 to 150,000 x g. The centrifugation may be performed at about 50,000 x g, 75,000 x g, 100,000 x g, 125,000 x g, or 150,000 x g.

In another embodiment, the sample of endometrial fluid comprising free and extracellular vesicle-associated miRNAs is subjected to a step of enrichment in extracellular vesicles prior to the determination of the levels of the miRNAs.

In one embodiment, the EVs are enriched in the sample according to the present invention have at the most 95%, at the most 90%, at the most 85%, at the most 80%, at the most 75%.at the most 70%, at the most 65%, at the most 60%, at the most 55%, at the most 45%, at the most 40%, at the most 35%, at the most 30%, at the most 25%, at the most 20%, at the most 15%, at the most 10%, at the most 5 %, at the most 4%, at the most 3%, at the most 2%, at the most 1% of the initial EVs concentration.

The term enriched "increased content of" or "increased level of" is meant a level of which is of by about 5 percent, about 10 percent, about 15 percent, about 20 percent, about 25 percent, about 30 percent, about 35 percent, about 40 percent, about 45 percent, about 50 percent, about 55 percent, about 60 percent, about 65 percent, about 70 percent, about 75 percent, about 80 percent, about 85 percent, about 90 percent, about 95 percent, about 100 percent, about 150 percent, about 200 percent, about 300 percent, about 400 percent, about 500 percent of the content of extracellular vesicles in the sample before enrichment step, or by more than 1.2-fold, about 1.4-fold, about 1.5-fold, about 1.8-fold, about 2.0-fold, about 3.0-fold, about 3.5-fold, about 4.5-fold, about 5.0-fold, about 10-fold, about 15-fold, about 20-fold, about 30-fold, about 40-fold, about 50-fold, about 100-fold, about 1,000-fold, or more of the content of extracellular vesicles in the sample before enrichment step.

The term "enriched for a surface marker" used herein is understood to refer to EVs that contain a marker that is present in the EVs (on the surface or in the interior) in a large amount or a high concentration, as compared with that in other EVs or in other membrane preparations used as a reference. That is, any marker is present inside or on the surface of the EVs and, therefore, if an EV may be distinguished from one or more other EVs or membrane preparations by using the marker, the EV preparation is considered be positive for the marker. Further, the term "positive" means that EVs have signals of higher intensity than a background intensity, for example, EVs have the marker in an amount enough to be detectable in a cell-measuring device.

EVs may have a diameter (or largest dimension where the particle is not spheroid) of between about 10 nm to about 5,000 nm (e.g., between about 50 nm and 1500 nm, between about 75 nm and 1,500 nm, between about 75 nm and 1250 nm, between about 50 nm and 1,250 nm, between about 30 nm and 1000 nm, between about 50 nm and 1,000 nm, between about 100 nm and 1000 nm, between about 50 nm and 750 nm, between 100 and 250 nm of about 200 nm).

In some embodiments, the EVs of the present invention are provided in the form of a preparation containing isolated EVs. As used herein, the terms "isolate," "isolated", "isolating", "purify", "purified" or "purifying" as well as "extracted" and "extracting" are used interchangeably and refer to the state of a preparation (e.g., a plurality of known or unknown amount and/or concentration) of EVs from endometrial fluid, that have undergone one or more processes of purification, e.g., a selection or an enrichment of the desired EV.

In another embodiment, the reference value for each miRNA is the level of said miRNA determined in a sample of endometrial fluid from a receptive endometrium.

In another embodiment, the levels of the miRNAs obtained in step (i) are normalized using the levels of at least one additional miRNA.

In another embodiment, the normalization is carried out using the levels of hsa-miR-200c-3p, of hsa-miR-92a-3p or of a combination thereof.

The term "hsa-miR-200c-3p", as used herein, relates to the human miRNA as defined in the mIRBase database of the University of Manchester (http://www.mirbase.org/index.shtml) (release 21 as of April 2021) under accession number MIMAT0000617, as well as to homologs and orthologues thereof (>hsa-miR-200c-3p; MIMAT0000617; UAAUACUGCCGGGUAAUGAUGGA - SEQ ID NO:5).

The term "hsa-miR-92a-3p", as used herein, relates to the human miRNA as defined in the mIRBase database of the University of Manchester (http://www.mirbase.org/index.shtml) (release 21 as of April 2021) under accession number MIMAT0000092, as well as to homologs and orthologues thereof (>hsa-miR-92a-3p; MIMAT0000092; UAUUGCACUUGUCCCGGCCUGU - SEQ ID NO:6).

### 2. In vitro method for selecting a female subject as a candidate to receive a therapy to increase or restore endometrial receptivity

The authors have found that determining the level of the miRNAs from a miRNA signature which is selected from the group consisting of miRNA signature a) comprising hsa-miR-24-3p, hsa-miR-200b-3p and/or hsa-miR-148b-3p miRNAs, and miRNA signature b) comprising hsa-miR-24-3p, hsa-miR-200b-3p and/or hsa-miR-99b-5p miRNAs allows for the selection of a female subject as a candidate to receive a therapy to increase or restore endometrial receptivity. The expression "determining the endometrial receptivity" as used herein relates to the prediction of a physiological or clinical status of the endometrium in regards to the pregnancy outcome (e.g. likelihood of pregnancy or implantation-capable embryo survival). The identification of a subject whose endometrium is receptive or not receptive provides with the basis for a clinical decision on how to proceed in assisted reproduction approaches. Said clinical decision may involve the treatment of the subject whose endometrium displays low receptivity to increase or restore endometrial receptivity.

Thus, in a second aspect, the invention relates to *in vitro* method (second method of the invention) for selecting a female subject as a candidate to receive a therapy to increase or restore endometrial receptivity which comprises
a. determining the endometrial receptivity of said subject by following the first method of the invention, and
b. selecting a subject as a candidate to receive an adequate therapy to increase or restore endometrial receptivity if the endometrium of the subject is not receptive.

The term "therapy", as used anywhere herein comprises any type of treatment, which aims at terminating, preventing, ameliorating and/or reducing the susceptibility to a clinical condition as described herein. In a preferred embodiment, the term therapy relates to prophylactic treatment (i.e. a therapy to reduce the susceptibility of a clinical condition, a disorder or condition as defined herein).

Thus, "therapy", "treatment" and the like, as used herein, refer to obtaining a desired pharmacologic and/or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "therapy" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter, such as inflammation, pain, and/or immune deficiency.

The terms "prevent", "preventing" and "prevention", as used herein, refer to a decrease in the occurrence of pathological cells in an animal. The prevention may be complete (e.g. the total absence of pathological cells in a subject). The prevention may also be partial, such that for example the occurrence of pathological cells in a subject is less than that which would have occurred without the present invention. Prevention also refers to reduced susceptibility to a clinical condition

The term "pharmacological treatment", "drug treatment", "drug therapy" and the like, as used herein, refers to a treatment comprising the administration of a drug to a subject to prevent, relieve and/or cure a disease, or to relieve, reduce or eliminate one or more symptoms associated with said disease, or to relieve, reduce or eliminate a risk factor for the progression of said disease, for example by treating a concomitant disease.

In a first step of the second method of the invention, the level of miRNA, particularly the miRNAs described previously in the context of the first method of the invention, is determined in a sample from a female subject for whom endometrial receptivity is to be determined. Methods to determine the level of a miRNA, particularly the miRNAs described previously in the context of the first method of the invention, are applicable to the second method of the invention. In a particular embodiment, miRNA levels are determined by RT-PCR. In another embodiment, miRNA levels are determined by miRNA microarray. The level of miRNA, particularly the miRNAs described previously in the context of the first method of the invention, wherein a difference between said level and a reference value is indicative of a receptive endometrium or is indicative of a low endometrial receptivity. Once a reference value of is established, the expression levels of circulating miRNAs expressed in a sample from the female subject whose endometrial receptivity is to be determined can be compared with this reference value, and thus be assigned a level of "increased" or "decreased", as indicated above in the context of the first method of the invention.

In the context of the second method of the invention, the terms "low endometrial receptivity" or "non-receptive endometrium" are used interchangeably. According to the previously described comparison of miRNA level and reference value in the context of the first method of the invention, the level of miRNA indicative of "non-receptive endometrium" is indicative of "low endometrial receptivity".

The terms "receptive endometrium" or "high endometrial receptivity", as opposed to "low endometrial receptivity" and used herein in relation to the level of a miRNA, particularly hsa-miR-24-3p, hsa-miR-200b-3p, hsa-miR-148b-3p and/or hsa-miR-99b-5p, relates to a situation where the level of hsa-miR-24-3p and/or hsa-miR-148b-3p is reduced at least 5%, at least 10%, at least 20%, at least 30%, at least 40%, at least 50%, at least 60%, at least 70%, at least 80%, at least 90% or at least 100% when compared to the corresponding reference value, and/or a level of hsa-miR-200b-3p is increased at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-24-3p is reduced at least 5%, at least 10%, at least 20%, at least 30%, at least 40%, at least 50%, at least 60%, at least 70%, at least 80%, at least 90% or at least 100% when compared to the corresponding reference value, and/or a level of hsa-miR-200b-3p and hsa-miR-99b-5p is increased at least 5%, at least 10%, at least 20%, at least 30%, at least 40%, at least 50%, at least 60%, at least 70%, at least 80%, at least 90% or at least 100% when compared to the corresponding reference value.

The term "low endometrial receptivity" used herein in relation to the level of a miRNA, particularly hsa-miR-24-3p, hsa-miR-200b-3p, hsa-miR-148b-3p and/or hsa-miR-99b-5p, relates to a situation where the level of hsa-miR-24-3p and/or hsa-miR-148b-3p is increased at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-200b-3p is reduced at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-24-3p is increased at least 5%, at least 10%, at least 20%, at least 30%, at least 40%, at least 50%, at least 60%, at least 70%, at least 80%, at least 90% or at least 100% when compared to the corresponding reference value, and/or the level of hsa-miR-200b-3p and/or hsa-miR-99b-5p is reduced at least 5%, at least 10%, at least 20%, at least 30%, at least 40%, at least 50%, at least 60%, at least 70%, at least 80%, at least 90% or at least 100% when compared to the corresponding reference value.

The term "receptive endometrium" as used herein relates to the situation in which the endometrium of the female subject shows at least 5%, at least 10%, at least 20%, at least 30%, at least 40%, at least 50%, at least 60%, at least 70%, at least 80%, at least 90%, at least 100% probabilities of acquiring a functional status that, upon transfer of a implantation-capable embryo, allows blastocyst adhesion and that will lead to pregnancy.

The term "low endometrial receptivity" as used herein relates to the situation in which the endometrium of the female subject shows at least 5%, at least 10%, at least 20%, at least 30%, at least 40%, at least 50%, at least 60%, at least 70%, at least 80%, at least 90%, at least 100% probabilities of not acquiring a functional status that, upon transfer of a implantation-capable embryo, allows blastocyst adhesion and that will lead to pregnancy.

As the person skilled in the art will understand, such determination does not usually seek to be correct for all (i.e., 100%) of the subjects that are going to be identified. However, the term requires that a statistically significant part of the subjects can be identified (for example, a cohort in a cohort study). The person skilled in the art can easily determine if a part is statistically significant using several well-known statistical evaluation tools, for example, determination of confidence intervals, determination of p values, Student's t-test, Mann-Whitney test, etc. The details are found in Dowdy and Wearden, Statistics for Research, John Wiley and Sons, New York 1983. The preferred confidence intervals are at least 90%, at least 95%, at least 97%, at least 98% or at least 99%. The p values are preferably 0.1, 0.05, 0.01, 0.005 or 0.0001. More preferably, at least 60%, at least 70%, at least 80% or at least 90% of the subjects of a population can be suitably identified by the method of the present invention.

Suitable samples to determine miRNA expression levels, particularly the levels of miRNAs described previously in the context of the first method of the invention, have been described previously in the context of the first method of the invention and are applicable to the second method of the invention.

In a second step of the second method of the invention, a subject is selected as a candidate to receive an adequate therapy to increase or restore endometrial receptivity if the subject has a low endometrial receptivity. The skilled person is aware of suitable therapies to increase or restore endometrial receptivity. By way of example and without limitation, the following strategies or therapies may be applied:
a) Cancelling the embryo transfer and starting a new cycle with a different endometrial preparation protocol;
b) Applying intrauterine therapy in the same cycle (washing of the endometrial cavity, intrauterine instillation of hormones or other drugs, infusion of specific culture media into the uterus);
c) Cancelling the embryo transfer and starting a new cycle, applying some intrauterine therapy in the new cycle, either days before the embryo transfer (washing of the endometrial cavity, intrauterine instillation of hormones or other drugs, infusion of specific culture media into the uterus, endometrial scratching), or immediately before the embryo transfer (washing of the endometrial cavity, intrauterine instillation of hormones or other drugs, infusion of specific culture media into the uterus).

In a particular embodiment, the subject is selected as candidate to receive an adequate therapy to increase or restore endometrial receptivity within the same *in vitro* fertilization cycle in which the sample from the subject has been obtained.

Embodiments disclosed in the context of the first method of the invention are also contemplated for the second method of the invention. In some embodiments, the sample is endometrial fluid. In some embodiments, said sample of endometrial fluid comprises free and extracellular vesicle-associated miRNAs. In some embodiments, prior to step i) according to the first method of the invention, said method comprises a non-invasive step of collecting at least one sample of endometrial fluid comprising free and extracellular vesicle-associated miRNAs. In some embodiments, the sample of endometrial fluid comprising free and extracellular vesicle-associated miRNAs is subjected to a step of enrichment in extracellular vesicles prior to the determination of the levels of the miRNAs. In some embodiments, the reference value for each miRNA is the level of said miRNA determined in a sample of endometrial fluid from a receptive endometrium. In some embodiments, the levels of the miRNAs obtained in step (i) according to the first method of the invention are normalized using the levels of at least one additional miRNA. In some embodiments, the normalization is carried out using the levels of hsa-miR-200c-3p, of hsa-miR-92a-3p or of a combination thereof. In some embodiments, the female subject is undergoing an in vitro fertilization procedure.

### 3. Method for achieving pregnancy in a woman

The authors have found that determining the level of the miRNAs from a miRNA signature which is selected from the group consisting of miRNA signature a) comprising hsa-miR-24-3p, hsa-miR-200b-3p and/or hsa-miR-148b-3p miRNAs, and miRNA signature b) comprising hsa-miR-24-3p, hsa-miR-200b-3p and/or hsa-miR-99b-5p miRNAs makes possible achieving pregnancy in a woman undergoing assisted reproduction procedures. The expression "determining the endometrial receptivity" as used herein relates to the prediction of a physiological or clinical status of the endometrium in regards to the pregnancy outcome (e.g. likelihood of pregnancy or implantation-capable embryo survival). The identification of a subject whose endometrium is receptive or not receptive provides with the basis for a clinical decision on how to proceed in assisted reproduction approaches. Said clinical decision involves either proceeding with embryo transfer if the endometrium is receptive or rather cancelling embryo transfer if the endometrium is not receptive or displays low receptivity. Therefore, the invention also relates to methods of achieving pregnancy in subjects for which the status of the endometrium is to be determined with the methods of the invention.

As used herein the term "pregnancy" is understood as the visualization of a gestational sac 4 week after embryo transfer. The term pregnancy may also be understood as the result of the final result of a fertilization event. "Pregnancy outcome" may be understood as the likelihood of the pregnancy after transferring an embryo into a receptive endometrium. The term "pregnancy outcome" also refers to a high implantation rate of embryo leading to pregnancy. The term "high implantation rate" means the potential of the embryo when transferred in the uterus, to be implanted in the uterine environment and to give rise to a viable foetus, which in turn develops into a viable offspring absent a procedure or event that terminates said pregnancy. The term "pregnancy outcome" also refers to endometrial receptivity, absent of implantation failure and early embryo miscarriage.

The expression "method for achieving pregnancy", as used herein, refers to any kind of measure either prophylactic or therapeutic, including means of any class, such as hygienic means, pharmacological means, surgical means or physical means, with the purpose of promoting pregnancy in a woman.

Thus, in a further aspect, the invention relates to a method for achieving pregnancy in a woman (third method of the invention) which comprises the steps of:
i) determining the endometrial receptivity of said woman by following the method according to the first method of the invention, and
ii) transferring an embryo in said woman if the endometrium of said woman is receptive.

In a first step of the third method of the invention, the level of miRNA, particularly the miRNAs described previously in the context of the first method of the invention, is determined in a sample from a female subject for whom endometrial receptivity is to be determined. Methods to determine the level of a miRNA, particularly the miRNAs described previously in the context of the first method of the invention, are applicable to the second method of the invention. In a particular embodiment, miRNA levels are determined by RT-PCR. In another embodiment, miRNA levels are determined by miRNA microarray. The level of miRNA, particularly the miRNAs described previously in the context of the first method of the invention, wherein a difference between said level and a reference value is indicative of a receptive endometrium or is indicative of low endometrial receptivity. Once a reference value of is established, the levels of miRNAs in a sample from the female subject whose endometrial receptivity is to be determined can be compared with this reference value, and thus be assigned a level of "increased" or "decreased", as indicated above in the context of the first method of the invention.

In the context of the second method of the invention, the terms "low endometrial receptivity" or "non-receptive endometrium" are used interchangeably. According to the previously described comparison of miRNA level and reference value in the context of the first method of the invention, the level of miRNA may be either indicative of a "receptive endometrium" or rather indicative of a "non-receptive endometrium".

The terms "receptive endometrium", "non-receptive endometrium", "endometrium is receptive", "endometrium is not receptive" have been defined in the context of the first method of the invention.

Suitable samples to determine miRNA expression levels, particularly the levels of miRNAs described previously in the context of the first method of the invention, have been described previously in the context of the first and second method of the invention and are applicable to the third method of the invention.

In a preferred embodiment, wherein the transferring of the embryo is carried out in the same in vitro fertilization cycle in which the sample in which the endometrial receptivity is determined is obtained.

Embodiments disclosed in the context of the first method of the invention are also applicable to the third method of the invention. In some embodiments, the sample is endometrial fluid. In some embodiments, said sample of endometrial fluid comprises free and extracellular vesicle-associated miRNAs. In some embodiments, prior to step i) according to the first method of the invention, said method comprises a non-invasive step of collecting at least one sample of endometrial fluid comprising free and extracellular vesicle-associated miRNAs. In some embodiments, the sample of endometrial fluid comprising free and extracellular vesicle-associated miRNAs is subjected to a step of enrichment in extracellular vesicles prior to the determination of the levels of the miRNAs. In some embodiments, the reference value for each miRNA is the level of said miRNA determined in a sample of endometrial fluid from a receptive endometrium. In some embodiments, the levels of the miRNAs obtained in step (i) according to the first method of the invention are normalized using the levels of at least one additional miRNA. In some embodiments, the normalization is carried out using the levels of hsa-miR-200c-3p, of hsa-miR-92a-3p or of a combination thereof. In some embodiments, the female subject is undergoing an in vitro fertilization procedure.

### 4. Method for increasing endometrial receptivity in a woman

The authors have found that determining the level of the miRNAs from a miRNA signature which is selected from the group consisting of miRNA signature a) comprising hsa-miR-24-3p, hsa-miR-200b-3p and/or hsa-miR-148b-3p miRNAs, and miRNA signature b) comprising hsa-miR-24-3p, hsa-miR-200b-3p and/or hsa-miR-99b-5p miRNAs of the invention allows for the development of a method for increasing the endometrial receptivity of a woman. The expression "determining the endometrial receptivity" as used herein relates to the prediction of a physiological or clinical status of the endometrium in regards to the pregnancy outcome (e.g. likelihood of pregnancy or implantation-capable embryo survival). The identification of a subject whose endometrium is receptive or not receptive provides with the basis for a clinical decision on how to proceed in assisted reproduction approaches, including non-surgical, surgical and therapeutical methods. According to the authors, said clinical decision involves the treatment of the subject whose endometrium displays low receptivity so as to increase or restore endometrial receptivity in said subject. Therefore, the invention also relates to methods for increasing endometrial receptivity in a woman who is selected as a candidate to receive a therapy based on the status of the endometrium to be determined with the methods of the invention.

Thus, in a fourth aspect, the invention relates to a method for increasing endometrial receptivity in a woman (fourth method of the invention) which comprises the steps of
i) determining the endometrial receptivity of said woman by following the method according to the first method of the invention,
ii) selecting a woman having low endometrial receptivity based on step i), and
iii) treating said woman with a therapy adequate to increase or restore endometrial receptivity.

In the first step of the fourth method of the invention, the endometrial receptivity of said woman is determined by using the first method of the invention. In the second step of the fourth method of the invention, a woman having low endometrial receptivity is selected based on step i). Said steps have been explained in the context of the first and second method of the invention and find application in the fourth method of the invention. Embodiments disclosed in the context of the first method of the invention are also applicable to this method of treatment.

The expression "method for increasing endometrial receptivity", as used herein, refers to any kind of measure, either prophylactic (to avoid the development of any physiological disorder or medical complications of a disease) or therapeutic (to treat said physiological disorder or complications when they are present), that a woman, whose endometrial receptivity is to be determined, undergoes with the purpose to increase endometrial receptivity and, in turn, facilitate or promote pregnancy. Said "method for increasing endometrial receptivity" includes means of any class, such as hygienic means, pharmacological means, non-surgical or surgical means, or physical means, with the purpose to increase or improve endometrial receptivity so that an implantation-capable embryo can, upon transfer into the uterus, successfully implant.

Methods for increasing endometrial receptivity depend on several factors such as the underlying physiological disorder or pathology, the family history and to some extent the individual morphology of each patient. Recommendations and criteria for election of treatment are known to the skilled person. By way of example and without limitation, the following strategies or therapies could be applied:
a) Cancelling the embryo transfer and starting a new cycle with a different endometrial preparation protocol;
b) Applying intrauterine therapy in the same cycle (washing of the endometrial cavity, intrauterine instillation of hormones or other drugs, infusion of specific culture media into the uterus);
c) Cancelling the embryo transfer and starting a new cycle, applying some intrauterine therapy in the new cycle, either days before the embryo transfer (washing of the endometrial cavity, intrauterine instillation of hormones or other drugs, infusion of specific culture media into the uterus, endometrial scratching), or immediately before the embryo transfer (washing of the endometrial cavity, intrauterine instillation of hormones or other drugs, infusion of specific culture media into the uterus).

In a particular embodiment, the subject is selected as candidate to receive an adequate therapy to increase or restore endometrial receptivity within the same in vitro fertilization cycle in which the sample from the subject has been obtained.

Embodiments disclosed in the context of the first method of the invention are also applicable to the fourth method of the invention. In some embodiments, the sample is endometrial fluid. In some embodiments, said sample of endometrial fluid comprises free and extracellular vesicle-associated miRNAs. In some embodiments, prior to step i) according to the first method of the invention, said method comprises a non-invasive step of collecting at least one sample of endometrial fluid comprising free and extracellular vesicle-associated miRNAs. In some embodiments, the sample of endometrial fluid comprising free and extracellular vesicle-associated miRNAs is subjected to a step of enrichment in extracellular vesicles prior to the determination of the levels of the miRNAs. In some embodiments, the reference value for each miRNA is the level of said miRNA determined in a sample of endometrial fluid from a receptive endometrium. In some embodiments, the levels of the miRNAs obtained in step (i) according to the first method of the invention are normalized using the levels of at least one additional miRNA. In some embodiments, the normalization is carried out using the levels of hsa-miR-200c-3p, of hsa-miR-92a-3p or of a combination thereof. In some embodiments, the female subject is undergoing an in vitro fertilization procedure.

### 5. Kit of the invention and uses thereof

The term "kit" or "kit-of-parts", as used herein, relates to an entity of physically separated components, which are intended for individual use, but in functional relation to each other. The kit of the invention is a product that contains the different reagents needed to carry on the different methods of the invention packed so as to allow their transport and storage. Materials suitable for packing the components of the kit include crystal, plastic (polyethylene, polypropylene, polycarbonate and the like), bottles, vials, paper, envelopes and the like. Additionally, the kits of the invention can contain instructions for the simultaneous, sequential or separate use of the different components which are in the kit. Said instructions can be in the form of printed material or in the form of an electronic support capable of storing instructions such that they can be read by a subject, such as electronic storage media (magnetic disks, tapes and the like), optical media (CD-ROM, DVD) and the like. Additionally or alternatively, the media can contain Internet addresses that provide said instructions.

The expression "reagent adequate for the determination of the level of a miRNA", as used herein, refers to any compound or set of compounds that allows the specific determination of the expression level of a miRNA by detection methods well known by the person skilled in the art.

The invention also relates to kits for carrying on the methods of the invention. Thus, in a fifth aspect, the invention relates to a kit comprising reagents adequate for the determination of the level of miRNAs forming part of a miRNA signature selected from the group consisting of a miRNA signature comprising or consisting of the hsa-miR-24-3p, hsa-miR-200b-3p and hsa-miR-148b-3p miRNAs, and a miRNA signature comprising or consisting of the hsa-miR-24-3p, hsa-miR-200b-3p and hsa-miR-99b-5p miRNAs.

In a preferred embodiment, the reagents adequate for the determination of the levels of said one or more miRNAs comprise at least 1%, at least 5%, at least 10%, at least 15%, at least 20%, at least 25%, at least 30%, at least 35%, at least 40%, at least 45%, at least 50% of the total amount of reagents forming the kit, preferably of the total amount of reagents adequate for the determination of the level of one or more miRNAs forming the kit. In further embodiments, the reagents adequate for the determination of the levels of one or more miRNAs comprise at least 55% at least 60%, at least 65%, at least 70%, at least 75%, at least 80%, at least 90%, at least 95%, at least 96%, at least 97%, at least 98% or at least 99% of the total amount of reagents forming the kit, preferably of the total amount of reagents adequate for the determination of the level of one or more miRNAs forming the kit.

In one embodiment, the kit comprises reagents adequate for the determination of the level of miRNA hsa-miR-24-3p. In another embodiment, the kit comprises reagents adequate for the determination of the level of miRNA hsa-miR-200b-3p. In another embodiment, the kit comprises reagents adequate for the determination of the level of hsa-miR-148b-3p. In another embodiment, the kit comprises reagents adequate for the determination of the level of hsa-miR-99b-5p. In another embodiment, the kit comprises reagents adequate for the determination of the level of hsa-miR-24-3p and hsa-miR-200b-3p. In another embodiment, the kit comprises reagents adequate for the determination of the level of hsa-miR-24-3p and hsa-miR-148b-3p. In another embodiment, the kit comprises reagents adequate for the determination of the level of hsa-miR-200b-3p and hsa-miR-148b-3p. In another embodiment, the kit comprises reagents adequate for the determination of the level of hsa-miR-24-3p, hsa-miR-200b-3p and hsa-miR-148b-3p.

In another embodiment, the kit comprises reagents adequate for the determination of the level of miRNAs hsa-miR-24-3p and hsa-miR-99b-5p. In another embodiment, the kit comprises reagents adequate for the determination of the level of hsa-miR-200b-3p and hsa-miR-99b-5p. In another embodiment, the kit comprises reagents adequate for the determination of the level of hsa-miR-24-3p, hsa-miR-200b-3p and hsa-miR-99b-5p.

In another embodiment, the kit comprises reagents adequate for the determination of the level of hsa-miR-24-3p, hsa-miR-200b-3p and a further miRNA selected from hsa-miR-148b-3p and hsa-miR-99b-5p.

In another embodiment, the kit comprises reagents adequate for the determination of the level of hsa-miR-24-3p, hsa-miR-200b-3p, hsa-miR-148b-3p and hsa-miR-99b-5p.

In a preferred embodiment, the reagents adequate for the determination of the level of said one or more miRNAs are oligonucleotides that hybridize specifically to said one or more miRNAs.

The term "oligonucleotide", as used herein, refers to a nucleic acid having preferably at least 10 nucleotides, preferably at least 15 nucleotides, preferably at least 20 nucleotides, preferably at least 25 nucleotides and preferably no more than 100 nucleotides, including polyribonucleotides, polydeoxyribonucleotides and combinations thereof. The term "oligonucleotide" also refers to molecules formed by conventional nucleotides bound by phosphodiester conventional bonds as well as variants thereof including modifications in purines or pyrimidines or modifications in riboses or deoxyriboses designed to increase the stability of the oligonucleotide. Alternatively or additionally, the oligonucleotides may contain modified bonds such as phosphotriester, phosphorothioate, methylphosphonate, etc, or may be peptide nucleic acids (PNA). Methods to synthesize oligonucleotides are well known by the person skilled in the art.

In a preferred embodiment, the oligonucleotides that hybridize specifically to hsa-miR-24-3p comprise at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 90%, at least 95%, at least 96%, at least 97%, at least 98% or at least 99% of the total amount of reagents forming the kit, preferably of the total amount of oligonucleotides forming the kit.

In a preferred embodiment, the oligonucleotides that hybridize specifically to hsa-miR-200b-3p comprise at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 90%, at least 95%, at least 96%, at least 97%, at least 98% or at least 99% of the total amount of reagents forming the kit, preferably of the total amount of oligonucleotides forming the kit.

In a preferred embodiment, the oligonucleotides that hybridize specifically to hsa-miR-148b-3p comprise at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 90%, at least 95%, at least 96%, at least 97%, at least 98% or at least 99% of the total amount of reagents forming the kit, preferably of the total amount of oligonucleotides forming the kit.

In a preferred embodiment, the oligonucleotides that hybridize specifically to hsa-miR-99b-5p comprise at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 90%, at least 95%, at least 96%, at least 97%, at least 98% or at least 99% of the total amount of reagents forming the kit, preferably of the total amount of oligonucleotides forming the kit.

In a preferred embodiment, the oligonucleotides that hybridize specifically to hsa-miR-24-3p, hsa-miR-200b-3p, hsa-miR-148b-3p and hsa-miR-99b-5p comprise, each of them, at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 90%, at least 95%, at least 96%, at least 97%, at least 98% or at least 99% of the total amount of reagents forming the kit, preferably of the total amount of oligonucleotides forming the kit.

These oligonucleotides are sequence-specific nucleic acids, such that they only quantify the sequence hybridizing with them and not all the double-stranded DNA. The oligonucleotide can be a primer or a probe.

Probes must be used for determining the level of RNA by means of PCR. A probe is an oligonucleotide having a defined sequence capable of specifically hybridizing with a complementary sequence of a nucleic acid, so it can be used for detecting and identifying complementary or substantially complementary sequences in nucleic acids. The length of the probe of the invention can vary within a large range although for practical reasons, probes having a small length of less than 10 nucleotides are preferred, preferably at least 15 nucleotides, preferably at least 20 nucleotides, preferably at least 25 nucleotides and, preferably not more than 100 nucleotides, more preferably comprised between 15 bases and 30 bases, preferably between 16 bases and 22 bases.

Alternatively or additionally, the oligonucleotides used can contain modified bonds such as phosphodiester, phosphotriester, phosphorothioate, phosphorodithioate, phosphoroselenoate, phosphorodiselenoate, phosphoroanilothioate, phosphoroamidate, methylphosphonate, boranophosphonate bonds, as well as combinations thereof, or they are peptide nucleic acids (PNAs), in which the different nucleotides are bound by amide bonds.

In case that the oligonucleotide is a probe, said probe can have at its 3' end a fluorophore and at its 5' end a molecule blocking its fluorescence emission (quencher). Said probe hybridizes specifically in the central part of the PCR product to be obtained.

In a related aspect, the present invention provides a set of one or more pairs of oligonucleotide primers designed to specifically amplify the miRNAs of the methods of the invention.

In a preferred embodiment, the present invention further provides a the present invention provides a kit containing a set of one or more pairs of oligonucleotide primers designed to specifically amplify the miRNAs of a miRNA signature in a reverse-transcriptase polymerase chain reaction assay, preferably a real-time quantitative reverse-transcriptase polymerase chain reaction assay.

In another embodiment, the present containing a microarray that has miRNA-specific probe oligonucleotides which specifically recognize the miRNAs identified as part of the miRNA signature of the invention. The microarrays contained in the kit may also include probe oligonucleotides which specifically recognize RNAs suitable for normalization purposes. In certain embodiments the kit further contains a control sample. This sample can be derived from a control or normal cell, tissue, or subject, e.g., one that exhibits, for example, normal traits. In additional embodiments, the kits of the invention contain instructions for use.

The expression "hybridize specifically" refers to such conditions that allow the hybridization of two polynucleotides of single chain, of complementary sequence or having a high grade of similarity, under stringent conditions or moderately stringent conditions, producing a double chain single molecule by base pairing.

"Stringency" of hybridization reactions are readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995). Stringent conditions and moderately stringent conditions are identified as described by Sambrook et al., Molecular Cloning: A Laboratory Manual, New York: Cold Spring Harbor Press, 1989.

The expression "reagent adequate for the determination of the level of expression a reference RNA", as used herein, refers to any compound or set of compounds that allows the specific determination of the level of a reference RNA by detection methods well known by the person skilled in the art.

In another embodiment, the kit further comprises comprising reagents adequate for the determination of the level of one or more reference RNAs and/or reagents adequate for the determination of the level of one or more constitutive genes.

The term "reference RNA" has been defined above in the context of the present invention. The term "constitutive gene", as used herein, refers to a housekeeping gene encoding a constitutively expressed protein and carrying out essential cell functions. Housekeeping genes suitable for use as internal standards include, but are not limited to, myosin, β-2-microglobulin, ubiquitin, 18S ribosomal protein, cyclophyllin, glyceraldehyde-3-phosphate dehydrogenase (GAPDH) and actin.

The kits of the invention also contain other reagents that allow determining the level of a miRNA but that are not specific for said miRNA, e.g. reagents for the extraction of RNA material, etc., e.g., primers for the synthesis of the corresponding cDNA by means of RT, reagents for the amplification of DNA such as DNA polymerases, dNTPs, buffers, etc.

In another aspect, the invention relates to the use of the kits according to the invention for determining endometrial receptivity, or for identifying a woman as a candidate to receive a therapy to increase endometrial receptivity.

In another aspect, the invention relates to the use of a kit of the invention for determining endometrial receptivity, or for identifying a woman as a candidate to receive a therapy to increase endometrial receptivity wherein the kit comprises reagents adequate for the determination of the level of one or more miRNAs selected from the group consisting of miRNA signature comprising the hsa-miR-24-3p, hsa-miR-200b-3p and hsa-miR-148b-3p miRNAs, and a miRNA signature comprising the hsa-miR-24-3p, hsa-miR-200b-3p and hsa-miR-99b-5p miRNAs.

### Additional aspects of the invention

1. An *in vitro* method for determining endometrial receptivity in a female subject which comprises the steps of:
   i) determining the level of the miRNAs from a miRNA signature, wherein said miRNA signature is selected from the group consisting of
      - a miRNA signature a) comprising the hsa-miR-24-3p, hsa-miR-200b-3p and/or hsa-miR-148b-3p miRNAs, and
      - a miRNA signature b) comprising the hsa-miR-24-3p, hsa-miR-200b-3p and/or hsa-miR-99b-5p miRNAs, in at least one sample from said subject, and
   ii) comparing the level of said miRNAs obtained in step (i) with a reference value for each of the miRNAs,
      wherein a reduced level of hsa-miR-24-3p and/or hsa-miR-148b-3p, and/or an increased level of hsa-miR-200b-3p, and/or an increased level of hsa-miR-99b-5p, with respect to the reference value, is indicative that the endometrium is receptive, and
      wherein an increased level of hsa-miR-24-3p and/or hsa-miR-148b-3p, and/or a reduced level of hsa-miR-200b-3p, and/or an increased level of hsa-miR-24-3p, and/or a reduced level of hsa-miR-200b-3p and/or hsa-miR-99b-5p,
      with respect to the reference value, is indicative that the endometrium is not receptive.
2. The method according to aspect 1, wherein
   signature a) comprises the hsa-miR-24-3p, hsa-miR-200b-3p and hsa-miR-148b-3p miRNAs; and
   signature b) comprises the hsa-miR-24-3p, hsa-miR-200b-3p and hsa-miR-99b-5p miRNAs, and
   wherein a reduced level of hsa-miR-24-3p and hsa-miR-148b-3p, and an increased level of hsa-miR-200b-3p, and/or a reduced level of has-miR-24-3p and an increased level of has-miR-200b-3p and hsa-miR-99b-5p, with respect to the reference value, is indicative that the endometrium is receptive, and
   wherein an increased level of hsa-miR-24-3p and hsa-miR-148b-3p, and a reduced level of hsa-miR-200b-3p, and/or an increased level of has-miR-24-3p and a reduced level of has-miR-200b-3p and hsa-miR-99b-5p, with respect to the reference value, is indicative that the endometrium is not receptive.
3. The method according to any one of aspects 1 or 2, wherein the levels of the miRNAs are indicative of receptivity within the same *in vitro* fertilization cycle in which the sample from the subject has been obtained.
4. An *in vitro* method for selecting a female subject as a candidate to receive a therapy to increase or restore endometrial receptivity which comprises
   a. determining the endometrial receptivity of said subject by following the method according to aspect 1, and
   b. selecting a subject as a candidate to receive an adequate therapy to increase or restore endometrial receptivity if the endometrium of the subject is not receptive.
5. The method according to any one of aspects 1 to 4, wherein the subject is selected as candidate to receive an adequate therapy to increase or restore endometrial receptivity within the same *in vitro* fertilization cycle in which the sample from the subject has been obtained.
6. The method according to any one of aspects 1 to 5, wherein the sample is endometrial fluid.
7. The method according to aspect 6, wherein said sample of endometrial fluid comprises free and extracellular vesicle-associated miRNAs.
8. The method according to any one of aspects 1 to 7, wherein prior to step i) said method comprises a non-invasive step of collecting at least one sample of endometrial fluid comprising free and extracellular vesicle-associated miRNAs.
9. The method according to any of aspects 6 to 8 wherein the sample of endometrial fluid comprising free and extracellular vesicle-associated miRNAs is subjected to a step of enrichment in extracellular vesicles prior to the determination of the levels of the miRNAs.
10. The method according to any one of aspects 1 to 9, wherein the reference value for each miRNA is the level of said miRNA determined in a sample of endometrial fluid from a receptive endometrium.
11. The method according to any one of aspects 1 to 10, wherein the levels of the miRNAs obtained in step (i) are normalized using the levels of at least one additional miRNA.
12. The method according to aspect 11, wherein the normalization is carried out using the levels of hsa-miR-200c-3p, of hsa-miR-92a-3p or of a combination thereof.
13. The method according to any one of aspects 1 to 12, wherein the female subject is undergoing an *in vitro* fertilization procedure.
14. Method for achieving pregnancy in a woman which comprises the steps of:
   i) determining the endometrial receptivity of said woman by following the method according to aspect 1, and
   ii) transferring an embryo in said woman if the endometrium of said woman is receptive.
15. The method according to aspect 14, wherein the transferring of the embryo is carried out in the same *in vitro* fertilization cycle in which the sample in which the endometrial receptivity is determined is obtained.
16. Method for increasing endometrial receptivity in a woman which comprises the steps of:
   i) determining the endometrial receptivity of said woman by following the method according to aspect 1,
   ii) selecting a woman having a non-receptive endometrium based on step i), and
   iii) treating said woman with a therapy adequate to increase or restore endometrial receptivity.
17. A kit comprising reagents adequate for the determination of the level of miRNAs forming part of a miRNA signature selected from the group consisting of a miRNA signature consisting of the hsa-miR-24-3p, hsa-miR-200b-3p and hsa-miR-148b-3p miRNAs, and a miRNA signature consisting of the hsa-miR-24-3p, hsa-miR-200b-3p and hsa-miR-99b-5p miRNAs.
18. The kit according to aspect 17, wherein the reagents adequate for the determination of the level of said one or more miRNAs are oligonucleotides that hybridize specifically to said one or more miRNAs.
19. The kit according to any one of aspects 17 or 18, further comprising reagents adequate for the determination of the level of one or more reference RNAs and/or reagents adequate for the determination of the level of one or more constitutive genes.
20. Use of a kit according to any one of aspects 17 to 19 for determining endometrial receptivity, or for identifying a woman as a candidate to receive a therapy to increase endometrial receptivity.

The invention is detailed below by means of the following examples which are merely illustrative and by no means limiting the scope of the invention.

### EXAMPLES

In detail, the authors of the present invention have developed a non-invasive method to identify the stage at which the endometrium of a female subject is receptive or prompt for embryo implantation, even in the case that the embryo transfer is performed in the same IVF cycle, by means of a miRNA signature. For this purpose, the authors have analyzed the expression of free and extracellular vesicle-associated miRNAs from the endometrial fluid of women in whom embryo implantation was successful and women in whom it was not. Currently, the molecular determination of endometrial receptivity is performed on endometrial biopsies. The latter is an invasive diagnostic method that prevents embryo transfer in the same cycle and, since it has a detrimental effect on implantation, it obliges to extrapolate the results obtained by analysis of the biopsy to the following cycle. For this reason, the current biopsy approach can be considered as a speculative procedure, since it is assumed that the results will be the same the following cycle, ignoring the fact that the endometrial cycle is a dynamic process in which many factors intervene that can influence the maintenance, acquisition or loss of endometrial receptivity. It is well known that obtaining endometrial fluid before the embryo transfer does not harm implantation. For this reason, the analysis of endometrial fluid obtained non-invasively, i.e. without the need for puncture or surgical intervention, constitutes a viable alternative to determine the ideal moment to perform the embryo transfer. In addition, endometrial fluid can be obtained several times during the cycle, and its analysis could predict whether the development of the endometrium will be ideal or whether changes, for example, the need of therapeutic intervention, will be required for efficient implantation.

In summary, this work demonstrates that it is possible to determine whether an endometrium of a female subject is receptive by analyzing EVs-associated miRNAs from a small volume of endometrial fluid samples obtained non-invasively from the female subject. This work also elucidates the specific EV-miRNAs as a miRNA signature which act as a biomarker for endometrial receptivity. The use of these biomarkers provides with a basis, in particular in female subjects undergoing IVF procedures, for a decision to proceed with embryo transfer in the same cycle as the cycle in which the sample was non-invasively collected from the female subject.

### Materials and Methods

### Experimental design

First, the optimization of sample collection and analysis from aspirated endometrial fluid (EF) samples was performed, as there is no protocol for the extraction of EVs from small volume of EF sample. Afterwards, a pilot study was carried out to set up a reproducible and sensitive methodology for analyzing EVs-associated-miRNAs from EF in clinical settings. For that, in order to assess the efficiency of each method seven miRNAs were analyzed by real-time quantitative PCR (RT-qPCR). Once the most efficient methods were selected, smallRNA sequencing (smallRNAseq) was performed to establish which of the procedures detected the largest number of miRNAs. Finally, the selected methodology was applied in a set of real samples with different implantation outcome to define a miRNA signature that allows the determination of which endometrium will be ready for implantation and which will not.

### Ethical approval

Ethical approval was obtained from the Cruces University Hospital Ethics Committee and Institutional Review Board (CEIC 11/45) and all of the participants gave written consent regarding their participation.

### Population

The population under study consisted of a cohort of 112 women who assisted at the Human Reproduction Unit of Cruces University Hospital (Basque Country, Spain) from January 2018 to August 2020. For the set up and optimization of the techniques, samples were collected from women who came to the examination room before starting any fertility treatment. Samples were obtained during natural cycle, 16-21 days after the beginning of the menstruation. For the third part of the study, the application of the selected method, the EF was obtained from a group of women undergoing frozen embryo transfer and the sample was collected immediately before the embryo transfer. Out of 112 women, 72 participate in the set up, and 40 participate in the validation of the selected methods. Of them, 15 achieved pregnancy and were included in the receptive endometrium group (implantative) and 25 did not achieve pregnancy and 15 were included in the non-receptive endometrium (non-implantative) group. Pregnancy was defined as visualization of a gestational sac 4 week after embryo transfer.

The inclusion criteria in this study were: i) age between 18 and 37 years, ii) cycle duration between 27 and 29 days, iii) absence of ovulatory disorders, myomas, endometriosis, polyps, uterine scars or hydrosalpinxs, iv) normal uterine and ovarian ultrasound v) normal hysterosalpingography and vi) absence of history of gynecological infections, immune disorders and of gynecological surgery. The inclusion criteria in the validation study also included i) frozen embryo transfer in day 5 and ii) transfer of 1-2 embryos resulting from owns oocytes. The substituted hormone treatment for endometrial preparation was a follicular rest control with transvaginal ultrasound in D1-D2 of the period. Hormone substitution treatment with 6 mg of oral estradiol (Progynova 2 mg/8 h), followed by serial ultrasound controls and when the endometrium reaches 7 mm, the day of the transfer is programmed (D+5). Start administering vaginal progesterone (Utrogestan 400 mg/ 12 hours), starting the morning of the day 1 (D+1) and making the transfer at D+5. Table 1 shows the main characteristics of the study population (mean ± SD).

### Sample collection and storage

Endometrial fluid was aspirated using a catheter used for embryo transfer (Frydman, Instrumentos Medicos Estériles SA, Spain) connected to a 10 mL syringe, under abdominal ultrasound guidance. Sample extraction was performed by gentle manual application of negative pressure with the syringe. To prevent contamination with cervical mucus, aspiration was interrupted at the internal cervical os. Special care was taken in the collection procedure to avoid touching the uterine fundus or injuring the cervix, and to minimize sample contamination with blood and endometrial tissue. In cases with excessive vaginal secretions, the vagina was cleaned with saline solution before the aspiration. Aspirate volumes ranged from 5 µL up to 50 µL and the samples were stored in two different ways according to their final purpose. In one case, the aspirated samples were directly expelled into standard cryogenic tubes and frozen at -80 °C until used. In the other case, after the aspiration, the 10 mL syringe was replaced with a 2 mL syringe containing 1.5 mL saline solution and the aspirates were mixed and expelled in a cryogenic tube (5-50 µL of EF + 1500 µL 1x DPBS (Gibco, Thermo Fisher Scientific #14190250). The mixed samples were centrifuged to remove contaminants at 2,000xg for 5 min and the supernatants were then frozen at -80 °C until processed. The supernatant samples dilution was 1:30 and they had a volume comprised between 400uL and 1300uL.

**Table 1. Main characteristics of the study population.**

| | **Implantative cycles (n=15)** | **Non-implantative cycles (n=15)** |
|---|---|---|
| Age, years (transfer) | 36.67 ± 2.58 | 36.33 ± 1.8 |
| Age, years (cryopreservation) | 35.52 ± 2.39 | 35.07 ±1,71 |
| Body mass index, kg/m2 | 26.17 ± 4.43 | 25.31 ± 1.92 |
| % Smokers | 26.7 | 25 |
| % Primary infertility | 78.6 | 73.3 |
| % Previous insemination failure | 40 | 41.6 |
| % Male factor | 37.5 | 26.7 |
| % Tubal factor | 6.7 | 0 |
| Estradiol at the day of hCG (pg / ml) | 3,580.14 ±1,820.23 | 4,101.4 ± 1,154.64 |
| Oocytes obtained | 13.47 ±5.22 | 15.6 ± 7.53 |
| MII oocytes | 11.73 ±5.07 | 13.2 ±5.71 |
| Fertilized oocytes | 7.21 ± 2.39 | 7.87 ±3.83 |
| Embryos obtained/conge | 3 ± 1.71 | 3.99 ± 3.97 |
| Embryos transferred | 1.33 ±0.49 | 1.33 ±0.49 |
| % Twins | 6.7 | |

### EV enrichment methods

### Size Exclusion Chromatography

A Poly-prep Chromatography Column (BioRad #731-1550) was filled with 2.5 mL of Sepharose CL-2B Cross-Linked resin (Sigma #CL2B300-100ML) and left packing overnight at 4°C. Then, the column was washed twice with 2.5 mL of 1 x DPBS. The sample of EF supernatant was applied into the column and once it had entered the column 4 mL of 1x DPBS were added. Fractions 1 to 10 had a final volume of 200 µL and fractions 11 and 12 had a final volume of 1 mL. The experiment was done in triplicates. The samples obtained from one of the replicas were used for analyzing the miRNAs among the fractions. The RNA corresponding to fractions 3 and 4 was analyzed using SmallRNAseq. The samples obtained from the other replicas were used for its characterization by WB.

### Polymer-based precipitation method (PBP)

There was not described any protocol to use Invitrogen Total Exosome Isolation Reagent with EF. Therefore, a protocol was optimized. 1) Centrifuge the EF supernatants at 3,000 g for 30 min at 4 °C. 2) Transfer the supernatants to a new tube and add 1 volume of the Total Exosome Isolation Reagent (1:1). 3) Mix the mixture gently by vortexing until there is a homogeneous solution. 4) After incubation, centrifuge the samples at 10,000 g for 1 hour at 4 °C. 5) Aspirate the supernatant by pipetting and discard. 6) The EVs are contained in the pellet, which may not visible, at the bottom of the tube. 7) Add 100 µL of 1x DPBS to resuspend the pellet. The resuspension volume could be modified according to the necessities of each case. µ

Both Total Exosome Isolation Reagent (from cell culture media #4478359) and Total Exosome Isolation Reagent (from other body fluids #4484453) worked well with EF. However, the inventors decided to use the #4478359 because it was cheaper and they were interested in defining a cost-effective protocol.

### Ultracentrifugation

Ultracentrifugation (UC) was carried out in a single step (100,000 xg for 75 min at 4°C) using a Beckman-Coulter TLA 120.2 rotor. EVs pellets were resuspended in 100 µL of 1x DPBS.

### RNA extraction methods

Total RNA isolation was performed by two different methods following the manufacturer's instructions. One of them was the mirVana^{™} PARIS^{™} Kit (MIR) (ThermoFisher #AM1556) and the other one was the Plasma/Serum RNA Purification Norgen kit (Norgenbiotek). In the second case, two different kits were used according with the necessities the midi kit (Norgen #56100) and the mini kit (Norgen #55000) (NOR). The RNA was eluted in nuclease free water (Ambion #AM9930), the resuspension volume were different according to the necessities of each case, and overall, the RNA was eluted in 50-100 µL. Of them, 2 µL were used for the cDNA synthesis and the rest was kept at - 80 °C until being used. All the analyses were done in triplicate.

### cDNA synthesis and Taqman miRNA assay

Following the manufacturer's recommendations cDNA was synthesized from 2 µL of RNA using TaqMan Advanced miRNA cDNA Synthesis kit (Applied Biosystems #A28007). The TaqMan reactions performed were TaqMan Fast Advanced Master Mix (ThermoFisher Scientific) and Taqman Advance miRNA assays (ThermoFisher Scientific). The qPCR was performed in a Via7 and the data was analyzed using the QuantStudio Real-Time PCR System software version 1.3 (Applied Biosystems). Two exogenous microRNAs were used as internal controls. To check differences in the efficiency of the RNA extraction 4 µL of cel-miR-39 (ThermoFisher 478293_mir) 0.1 nM were added to the sample before each RNA extraction procedure. To test differences during the cDNA synthesis process 0.2 µL of ath-miR-159a (ThermoFisher, 478411_mir) 0.001 nM were added at the beginning of each cDNA synthesis reaction. This miRNA was used to calculate the relative quantity of the analyzed miRNAs. The expression profile of seven EVs-associated-miRNAs were analyzed as reference miRNAs. These miRNAs have been previously described to be secreted by endometrial epithelial cell lines and to be present in EF aspirates. Of them, hsa-miR-200c-3p (ThermoFisher, 478351_mir), hsa-miR-17-5p (ThermoFisher, 478447_mir), hsa-miR-30c-5p (ThermoFisher, 478008_mir) hsa-miR-92a-3p (ThermoFisher, 477827_mir) and hsa-let-7e-5p (ThermoFisher, 478579_mir) are detected among the most abundant microRNAs, with hsa-miR-200c-3p, being the most abundant of all. The same authors have described hsa-miR-451a (ThermoFisher 478107_mir) to be selectively expressed in EVs. In addition, hsa-miR-30d-5p (ThermoFisher, 478606_mir) has also found to be secreted in endometrial exosomes and has been related with the early embryo implantation. In addition, other two miRNAs were analyzed, hsa-miR-21-5p (ThermoFisher, 477975_mir) was one of the most detected miRNA in the smallRNAseq of the setup, whereas hsa-miR-155-5p (ThermoFisher, 483064_mir) was among the least detected.

### DTT treatment

EF samples were treated with 1.4 % DTT solution in 1:1 portion. For the control 1x DPBS 1:1 was added. The samples were gently mixed by vortexing and incubated at room temperature for 15 min. Then 1x DPBS was added until the EF samples were diluted 1:8 and were centrifuged 3000 g for 15 min at 4 °C. The supernatants were recovered and 400 µL aliquots were done. After that, the EV enrichment was done with Total Exosome Isolation Reagent (ThermoFisher #4478359) and EVs were resuspended in 100 µL of 1x DPBS. Of them, 15 µL were reserved for western blotting (WB), 5 µL for Cryo electron microscopy, 5 µL for Nanoparticle Tracking Analysis (NTA) and the rest was used for RNA analysis with Norgen Plasma/Serum RNA Purification Mini kit (Norgen #55000). The isolated RNA was eluted in 100 µL of nuclease-free water. Of them 2 µL were used for the following cDNA synthesis and the rest was storage at -80 °C.

### RNase protection assay

EF samples were enriched in EVs using Invitrogen Total Exosome Isolation Reagent (ThermoFisher #4478359) with a final volume of 200 µL. Aliquots were treated following different procedures, RNase A (Sigma Aldrich # 10109142001) (RNase), Proteinase K (Sigma Aldrich #03115879001) + RNase (PRT-K), TX-100 (Sigma Aldrich #T8787) + RNase (TX-100), and TX-100 + Proteinase K + RNase (TX+PRT). In parallel, an untreated sample was carried out which was used as a control of the analysis. Samples were treated with 1µL TX-100 at 20%, to achieve a final concentration of 0.1% TX-100. The proteinase K was added in 0.05 mg/mL concentration and was incubated for 10 min at 37 °C. Then the reaction was stopped by adding 5 mM of phenylmethylsulfonyl fluoride (PMSF) (Sigma Aldrich #10837091001) and heating the samples at 90 °C for 5 min. Samples were finally treated with 0.1 mg/mL RNase A (RNase) for 20 mins at 37 °C. The control samples were kept at 4 °C until RNA extraction. Before the extraction, β-mercaptoethanol was used to inhibit the RNase as described in the Norgen Plasma/Serum RNA Purification Mini kit (Norgen #55000). The isolated RNA was eluted in 50 µL of nuclease-free water. Of them 2 µL were used for the following cDNA synthesis and the rest was storage at -80 °C. All the analyses were done in triplicate with two technical duplicates, ending with six TaqMan RT-qPCR replicates.

### Electron Microscopy

For cryo-electron microscopy, EV preparations were directly adsorbed onto glow-discharged holey carbon grids (QUANTIFOIL, Germany). Grids were blotted at 95% humidity and rapidly plunged into liquid ethane with the aid of VITROBOT (Maastricht Instruments BV, The Netherlands). Vitrified samples were imaged at liquid nitrogen temperature using a JEM-2200FS/ CR transmission cryo-electron microscope (JEOL, Japan) equipped with a field emission gun and operated at an acceleration voltage of 200 kV.

### SmallRNA sequencing

The quantity and quality of the RNAs were evaluated using Agilent RNA 6000 Pico Chips (Agilent Technologies, Cat. # 5067-1513). Sequencing libraries were prepared following the protocol included with the kit "NEXTflex^{™} Small RNA-Seq Kit v3," (^{©}Bioo Scientific Corp. Catalog #5132-06, protocol V19.01). Briefly, total RNA of each sample was incubated for 2 minutes at 70°C, then 3' 4N adenylated adapter (adapter dilution 1/4) and ligase enzyme were added and ligation was conducted by incubation of this mix at overnight at 20°C. After excess 3' adapter removal, 5'-adapter was added alongside with ligase enzyme and the mix was incubated at 20°C for 1 hour. The ligation product was used for the reverse transcription with the M-MuLV Reverse Transcriptase in a thermocycler for 30 min at 42°C and 10 min 90°C. Next, enrichment of the cDNA was performed using PCR cycling: 2 min at 95°C; 20-27 cycles of 20 sec at 95 °C, 30 sec at 60 °C and 15 sec at 72°C; a final elongation of 2 min at 72°C and pause at 4°C. PCR products were resolved on 8% Novex TBE PAGE gels (Cat. # EC6215BOX, Thermo Fisher Scientific), and one band between 150 bp and 400 bp was cut from the gel. SmallRNAs were extracted from polyacrylamide gel using an adapted protocol, in which DNA from gel slices was dissolved in ddH2O overnight at room temperature. Afterwards, libraries were visualized on an Agilent 2100 Bioanalyzer using Agilent High Sensitivity DNA kit (Agilent Technologies, Cat. # 5067-4626) and quantified using Qubit dsDNA HS DNA Kit (Thermo Fisher Scientific, Cat. # Q32854). Qualified libraries were sequenced in a HiSeq2500 (Illumina Inc) to get at least 10 million 50nt-single-reads per sample.

### Data analysis

### Real-Time quantitative PCR (RT-qPCR) assay

The relative expression levels of the miRNAs analyzed in the setup were normalized to ath-miR-159 and were calculated using the 2-ΔCT (Ct miRNA - Ct ath-miR-159a) method (Livak et al., Methods 2001; 25(4):402-408). The relative expression levels of the validated miRNAs were normalized to the selected "hsa-miR-200c and hsa-miR-92a" and were calculated using 2-ΔΔCT method (Rao et al., Biostat Bioinforma Biomath 2013 Aug;3(3):71-85). NormFinder software was used to determine the normalization miRNAs.

### SmallRNA sequencing data

FASTQs were trimmed for the adapters following the manufacturer's recommendations of the NEXTflex^{™} Small RNA-Seq kit. Bowtie was used to align reads against the human genome with the corresponding annotations (GRCh38/GENCODE-v26). GENCODE includes a full set of annotations including all protein-coding loci with alternatively transcribed variants, non-coding loci with transcript evidence, and pseudogenes. Quantification to the transcriptome was performed by Partek expectation maximization (EM) using the Partek Flow software version 7.0. Only those miRNAs that presented 10 or more reads in the three replicas were considered. To visualize the abundance of each miRNA, the raw counts were normalized to the mean number of counts for each sample and represented using the pheatmap package v1.0.10 (default settings) in R 3.4.1 software (2014-04-10, R Foundation for Statistical Computing, Vienna, Austria). UpSet plot showing the total size and overlaps between the miRNAs sets isolated by each method or SEC fraction was performed using UpSetR (available online in https://gehlenborglab.shinyapps.io/upsetr/).

### Alignment

FASTQs were trimmed for the adapters following the recommendations of the NEXTflex^{™} Small RNA-Seq Kit manufacturers. We used Bowtie (B. Langmead, et al., Genome Biol. 10, R25 (2009)) to align the reads against the human genome (GRCh38), with a mismatch 0 to avoid false positives. We selected mirbase v22 to quantify mature miRNAs employing Partek Flow application, software version 7.0.

### SmallRNAseq data analysis

We performed differential abundance analyses to discover miRNAs associated with implantation outcome. To avoid molecules with sparse presence, following TMM normalization we retained miRNAs with (i) counts per million (CPM)>1, (ii) non-zero counts in at least 15 individuals, and (iii) at most 10 zero counts in each of the two subgroups, namely women in whom the implantation was successful (n=15) and those in which it was not successful (n=15). Differential expression was then assessed in edgeR (M. D. Robinson, et al., Bioinformatics. 26, 139-140 (2009)) using the SARTools R package (H. Varet, et al., PLoS One. 11, 1-8 (2016). Benjamini-Hochberg procedure was used to calculate the false discovery rate for each comparison and obtain adjusted p-values.

### Regression study

A subset of miRNAs was used to generate two linear regression models with k-fold cross-validations, one per each miRNA extraction protocol assessed. Samples were randomly divided into training and testing datasets (80-20%). Three miRNAs were used per each modelling process, selected hsa-miR-24-3p, hsa-miR-200b-3p and hsa-miR-148b-3p for PBP-M and hsa-miR-24-3p, hsa-miR-200b-3p and hsa-miR-99b-5p for PBP-N. Resulting models reproducibility was further tested by bootstrap correction, with 500 replications. All the analysis was done on R v4.0.0 software (R Development Core Team; http://cran.r-project.org) with ROCR (42) and caTools packages.

### Correlation analysis

Correlation coefficients between qPCR and smallRNAseq data were calculated using the cor function in R 3.4.1 software.

### Results

### Characterization of the endometrial fluid (EF)-derived extracellular vesicles (EVs)

In the clinic, when the sample is aspirated with the catheter, it is necessary to use 1.5 mL of PBS to expel the entire sample from the catheter as it get stuck in its walls. After the centrifugation, the supernatant is collected and a non-dissolved mucus pellet remains at the bottom. To determine whether the extracellular vesicles (EVs) were released into the PBS and were not trapped in the mucous, an endometrial fluid (EF) pool (EFP) was made and it was divided in two aliquots. One of the aliquots was solubilized with 1.4% DTT, whereas the other was mixed with the same volume PBS without DTT. It was observed that when the EF was treated with DTT most of the mucus was degraded. The analyses with the Nanoparticle Tracking Analysis (NTA) and the cryo-electron microscopy (CryoEM) revealed heterogeneous EV populations with diameters between 100 and 800 nm in both conditions. However, there were bigger size particles in the supernatant of the DTT positive samples in comparison with those observed in the untreated group. In the case of untreated samples, the average of the particles detected was 1.94 x 109 ± 7.80 x 107 particles/mL. The mean of the particle size was 291.5 ± 0.1 nm and the mode was 196.4 ± 3.2 nm, although particles of larger size could also be detected. In the samples treated with DTT, it was possible to detect more particles 2.70 x 109 ± 5.86 x 107 particles/mL, and also, different subpopulations. In addition, in the treated samples, the particles were larger, the mean was 313.6 ± 2.5 nm and the mode was 248.5 ± 8.1 nm. Western blotting (WB) analysis showed different pattern of vesical markers among the conditions. The Rab8 marker was more intense in the treated samples, which correlated with the number of particles detected in the NTA. In contrast, Limp II, CD133 and CD63 markers were more intense in the untreated samples. Real time quantitative polymerase chain reaction (RT-qPCR) analysis was used to quantify the selected reference miRNAs in the samples.

### Analysis of EVs-associated-miRNAs from endometrial fluid

A comparative analysis of five different methodologies was performed in order to define a simple and effective strategy for detecting EVs-associated-miRNAs from endometrial fluid (EF). An EFP was done with frozen EF supernatants. All the analyses were done in triplicate with two cDNA synthesis duplicates, ending with 12 TaqMan RT-qPCR replicates. The recovery volumes of the supernatants varies among the sample due to many factors such as the person collecting the sample, the volume or the viscosity of the EF. In general, the supernatants ranged from 400 µL to 1.3 mL. Therefore, the protocols were optimized to be used with the minimum volume available that was 400 µL in one of the samples collected. Although it is always best to use the maximum amount of sample possible, the goal of this experiment was to allow its use in the majority of the cases. RNA extraction methods mirVana (DCT-M) and Norgen (DRC-N), two of the compared protocols, were designed to extract RNA directly from the endometrial fluid (EF), the difference between them was the extraction kit used in each case. The other three protocols included an initial EVs enrichment step followed by a RNA extraction procedure. An ultracentrifugation step (ULT) before RNA extraction was performed with mirVana. PBP-N and PBP-M had an EV-enrichment procedure with Invitrogen commercial kit followed by RNA extraction with Norgen and mirVana respectively.

To determine the efficiency of each protocol seven reference miRNAs were analyzed using RT-qPCR. All the miRNAs analyzed could be detected in all the replicas of the five techniques. However, the differences resided in the amount of RNA detected for each of the miRNAs. The efficiency analysis showed that the protocols with a previous enrichment step in EVs with Invitrogen Total Exosome Isolation Reagent performed better in comparison with the rest. PBP-N was by far the most efficient method. The methodologies with a direct extraction (DCT-N and DCT-M) and ULT obtained less quantity of miRNAs.

To further explore the RNA extracted with PBP-N and PBP-M a small RNA sequencing (smallRNAseq) was performed. It was used to establish with which of the two methods it was possible to detect a greater number of miRNAs. In addition, RNA obtained from F3 and F4 of the size exclusion chromatography (SEC), which were associated with small-EVs, were also analyzed by smallRNAseq. Only those miRNAs that had at least 10 raw counts in the three replicas and appeared at least ten times more than in the blank sample were taken into account. The results showed that with PBP-M method 251 unique miRNAs could be detected, while with PBP-N only 151 miRNA could be detected. Among these 402 miRNAs, the two techniques shared 145. For F3 and F4 204 and 149 unique miRNAs respectively could be detected. Then, the miRNAs obtained through enrichment with INV and the miRNAs obtained with SEC were compared and it was seen that the great majority of them could be detected. The percentage of alignment with the human genome for each case were PBP-M 74.0 ± 5.87 %, PBP-N 68.4 ± 1.78 %, F3 54.0 ± 6.79 %, and F4 67.7 ± 5.14 %. These data show that with these two techniques (PBP-M and PBP-N) it is possible to detect most of the EVs-associated-miRNAs from EF.

Given the importance of selecting a robust methodology, the technical reproducibility of PBP-M and PBP-N was also investigated. For that, two people carried out a technical reproducibility experiment and a new EFP was done. Each person analyzed 20 aliquots, 10 of them using PBP-N and the other 10 with PBP-M. To test the reproducibility of the methodologies RT-qPCR was used to analyze the seven reference miRNAs and two other miRNAs obtained from the smallRNAseq results; hsa-miR-21-5p that was one of the most abundant miRNA and hsa-miR-155-5p that was one of the least abundant. The nine miRNAs could be detected with PBP-N independently of the person performing the EVs enrichment and the RNA extraction. However, using PBP-M it only was possible to detect has- hsa-miR-155-5p and miR-21-5p in 12/20 and 14/20 replicas respectively, although they were detected using *SmallRNAseq* in all the replicas. The coefficients of variation showed a greater variability for the results obtained with PBP-M, both among the different aliquots and among the people who performed the experiment. This was not the case with the results obtained with PBP-N. In addition, the normalized relative quantification of the amount of miRNAs detected showed significant differences between the techniques, being PBP-N the one that was able to detect more quantity of all miRNAs in comparison with PBP-M.

Overall, PBP-M and PBP-N obtained the best results from this optimization procedure. The PBP-M technique turned out to be more efficient since a greater number of miRNAs were detected using SmallRNAseq. However, PBP-N proved to be more efficient in terms of RT-qPCR detection. Since in all the experiments performed with this technique, all miRNAs analyzed could be detected with lower coefficients of variation.

Among the methodologies that were developed, PBP-M and PBP-N were chosen for its implementation in a set of individual samples, as they were the most efficient ones. In this case, supernatants of EF samples were collected from 15 women in whom the implantation had been successful and achieved a pregnancy and in other 15 that did not. There were no significant differences between the group characteristics (see Table 1). The volume of the samples ranged from 825 µL to 1400 µL, of which 800 µL were used for the comparison of the techniques, 400 µL were analyzed with PBP-N and another 400 µL were analyzed with PBP-M.

To further explore the total RNA extracted with each method the *SmallRNAseq* was used. With the results, a post-alignment QA/QC analysis was performed to assess the quality of the alignment task for each methodology. The alignment of the sequences against mirBase showed that with PBP-M it was possible to detect 845 unique mature miRNA sequences while with PBP-N it was possible to detect 910 mature miRNAs sequences. Of them, 832 were detected in both techniques, while 13 were specific for PBP-M and 78 for PBP-N. However, differences in the performance of the techniques were found as different results were found for the percentage of alignment and total unique miRNA detected in the same samples. The alignment percentage results for the EF_29 were 1.49% with PBP-M and 47.13% with PBP-N, and the number of unique detected miRNAs were 1 and 290 respectively.

The differences observed among the different samples were taken into account when statistical analyses were done. The following restriction was applied in order to decrease the influence of sample differences on the results. First, all the miRNAs that had less than 10 times more counts than the blank sample were removed. Then those samples that have no more than 100 miRNAs with at least 10 counts identified, which corresponds approximately to the samples that had less than the 20% of the total miRNAs detected, were eliminated from the analysis. In an attempt to reduce the number of miRNAs to be analyzed, only those that appeared at least in the 70% of the samples in each group and those that besides being in 70% of a group were in less than 50% of the samples of the other group were selected. After having applied the restrictions, 231 miRNAs with PBP-M and 341 miRNAs with PBP-N could be detected. Finally, the miRNAs selected for validation were the miRNAs with a p-adjusted value lower than 0.05 (p<0.05) and a fold change ± 1.5 against the other group.

When these criteria were applied in the PBP-M data, 13 miRNAs suitable for validation could be detected (see Table 2). With PBP-N method, only 5 miRNAs that met the criteria were detected, of which two were common with PBP-M (see Table 2). However, the raw counts before the normalization showed the same trend among the miRNAs detected by the PBP-M and the PBP-N, so it was decided to re-validate the 16 miRNAs in the same samples using RT-qPCR.

In order to perform the validation, the first critical step is to select an adequate endogenous control. For that, the variability of expression of various endogenous candidate controls in the EF were compared to determine their stability. First, the variability of the seven reference genes that were used before and the hsa-miR-21-5p, which was detected to be among the most abundant one, were determined in the smallRNAseq results. It was found that all of them could be detected in most of the samples with high row counts, and statistical analyses showed that there were not statistical differences among the groups. In addition, their stability was also tested by RT-qPCR. In this case, 400µL of EF were used from 40 individuals (30 of them were the same as in the small RNA seq experiment). The RNA extraction done by PBP-N and 2µL of RNA were used in each case. Of the seven reference miRNAs analyzed, only hsa-miR-200c-3p could be detected in all of the samples. The minimum CT value was 15.5 while the maximum was 31.58 giving a coefficient variation of 19.19%. Overall, the coefficient of variation of the rest of miRNAs varied between 13.44% (for the hsa-miR-30c-5p and 22.31 % (for the hsa-miR-21-5p). Normfinder algorithm was used to identify the optimal normalization miRNA and the best pair of normalizer miRNAs. The results showed that both hsa-miR-200c-3p (stability PBP-M= 0.31 and PBP-N= 0.24) and hsa-miR-92a-3p (stability PBP-M= 0.27 and PBP-N= 0.1) were suitable internal controls.

Once the endogenous controls were selected, the validation of the 16 miRNAs candidates was performed by RT-qPCR. The validation was performed in 20 out of the 30 samples analyzed by smallRNAseq that had good RT-qPCR amplification results when the determination of the internal control was done. The samples selected were EF_1, EF_3, EF_5, EF_6, EF_8, EF_9, EF_10, EF_12, EF_13, EF_30, EF_11, EF_17, EF_19, EF_20, EF_21, EF_25, EF_26, EF_27, EF_28, EF_29. The normalization of the results was done against the Ct values combination of hsa-miR-200c-3p and hsa-miR-92a-3p, which were determined as good internal controls.

Table 2 shows the list of miRNAs selected for validation by RT-qPCR in the same samples.

**Table 2. miRNAs selected for validation.**

| | | **PBP-M** | | **PBP-N** | |
|---|---|---|---|---|---|
| **miRNAs** | **Detection method** | **edgeR Log2FC** | **edgeR Adjusted p-value** | **edgeR Log2FC** | **edgeR Adjusted p-value** |
| hsa-miR-132-3p | PBP-M | -5.338 | 0.0002 | 0.365 | 0.2700 |
| hsa-miR-136-3p | PBP-N | -0.269 | 0.9000 | 3.036 | 0.0400 |
| **hsa-miR-148b-3p** | **PBP-M** | **3.719** | **0.0002** | **0.132** | **0.9600** |
| hsa-miR-185-5p | PBP-M | 3.332 | 0.0024 | -0.707 | 0.7400 |
| hsa-miR-196b-5p | PBP-M | -2.694 | 0.0006 | 0.159 | 0.9400 |
| **hsa-miR-200b-3p** | **PBP-M** | **1.492** | **0.0300** | **0.486** | **0.4400** |
| hsa-miR-214-5p | PBP-N | 0.961 | 0.4700 | -1.665 | 0.0073 |
| hsa-miR-218-5p | BOTH | 2.05 | 0.0300 | 1.193 | 0.0076 |
| hsa-miR-224-5p | PBP-M | 2.214 | 0.0300 | -0.118 | 0.8400 |
| hsa-miR-23a-3p | PBP-M | -1.702 | 0.0200 | 0.549 | 0.4400 |
| **hsa-miR-24-3p** | **PBP-M** | **-1.786** | **0.0400** | **0.046** | **0.5500** |
| hsa-miR-34c-5p | PBP-M | 2.266 | 0.0200 | -0.456 | 0.7300 |
| hsa-miR-3529-3p | PBP-M | 5.05 | 0.0002 | 0.713 | 0.2000 |
| hsa-miR-378a-3p | PBP-M | 2.916 | 0.0300 | 0.126 | 0.3600 |
| hsa-miR-425-5p | PBP-N | 1.112 | 0.2500 | -2.527 | 0.0300 |
| **hsa-miR-99b-5p** | **BOTH** | **-2.755** | **0.0004** | **0.156** | **0.0300** |

The results for the validation in samples analyzed with the PBP-M method showed that of the 16 miRNAs selected only the expression of 2 miRNAs was significantly different between the groups; hsa-miR-24-3p (p-value:0.0021) and hsa-miR-132-3p (p-value: 0.0032). Other three miRNAs followed the same trend that in smallRNseq and were almost significant (p-values 0.1-0.05): hsa-miR-196b-5p, hsa-miR-200b-3p and hsa-miR-34c-5p. Of them, hsa-miR-24-3p,-miR-132-3p and hsa-196b-5p were down regulated in the implantation group, while hsa-miR-200b-3p and hsa-miR-34c-5p appear to be up regulated in the implantation group. In addition, there were other 6 miRNAs that followed the same trend observed in smallRNAseq although they were not significant. On the contrary, hsa-miR-185-5p (p value: 0.012) and hsa-miR-148b-3p (p value: 0.147) were found to be down regulated by RT-qPCR in the implantation group, while in smallRNAseq were up regulated. With the rest of miRNAs inconsistencies were found between the results obtained with the internal controls and the results of the smallRNAseq.

The validation of the PBP-N technique showed that of the 16 miRNAs selected the expression of 2 miRNAs was significantly different between the groups, both being up regulated in the implantation group, hsa-miR-200b-3p (p-value:0.023) and hsa-miR-99b-5p (p-value: 0.022). There also were other 4 miRNAs up regulated in the implantation (hsa-miR-132, hsa-miR-23-3p, hsa-miR-3529 and hsa-miR-378a-3p) and one down regulated (hsa-miR-214-5p) without statistical differences. On the contrary, 7 out of 16 miRNAs did not follow the trend expected in *SmallRNAseq* (hsa-miR-24-3p, hsa-miR-185-5p, hsa-miR-136-3p, hsa-miR-196b-5p, hsa-miR-218-5p, hsa-miR-224-5p and hsa-miR-34-5p). Of them, hsa-miR-24-3p (p value: 0.013) was down regulated in the implantation group, and the result matches the result obtained with PBP-M. In the case of hsa-miR-185-5p, it was found to be up regulated in implantation group for PBP-N analysis, while, in the results of PBP-M it was found to be down regulated by RT-qPCR. With the hsa-425-5p and hsa-miR-148b-3p, inconsistencies were found between the results obtained with the internal controls and the results of the smallRNAseq. In addition, when the RT-qPCR results were compared among the methodologies 7 out of 16 miRNAs were found to follow the same patter. Of them, 4 were down regulated (hsa-miR-136-3p, hsa-miR-148b-3p, hsa-miR-196b-5p and hsa-miR-24-3p) in the implantation group and 3 were up regulated (hsa-miR-200b-3p, hsa-miR-34c-5p and hsa-miR-3529-5p).

The results for the validation of each miRNA listed in Table 2 are summarized in Table 3. The term PBP refers to the procedure performed with the commercial kit Invitrogen Total Exosome Reagent to enrich the samples in extracellular vesicles before RNA extraction. The term PBP-N refers to the RNA extraction method performed with the commercial kits mirVAna Paris kit. The term PBP-M refers to the RNA extraction method performed with the commercial kit Norgen kit. "Level" and "Fold Change" (FC) refer to the status of each miRNA in the implantation (receptive endometrium) group. Specifically, the term "UP" represents an increase of level of a given miRNA in the sample from a receptive endometrium with respect to the level of the same miRNA in a sample from a non-receptive endometrium. In contrast, the term "DOWN" indicates a reduction of level of a given miRNA in the sample from a receptive endometrium with respect to the level of the same miRNA in a sample from a non-receptive endometrium.

**Table 3. miRNAs validation by qRT-PCR**

| | **PBP-M** | | | **PBP-N** | | |
|---|---|---|---|---|---|---|
| **microRNA** | **Level** | **FC** | **p-value** | **Level** | **FC** | **p-value** |
| hsa-miR-132-3p | DOWN | 0.436 | ** 0.003 | UP | 1.098 | 0.754 |
| hsa-miR-136-3p | DOWN | 0.653 | 0.237 | DOWN | 0.664 | 0.186 |
| **hsa-miR-148b-3p** | **DOWN** | **0.751** | **0.148** | **DOWN** | **0.921** | **0.694** |
| hsa-miR-185-5p | DOWN | 0.522 | *0.012 | UP | 1.408 | 0.153 |
| hsa-miR-196b-5p | DOWN | 0.272 | 0.052 | DOWN | 0.551 | 0.307 |
| **hsa-miR-200b-3p** | **UP** | **1.418** | **0.088** | **UP** | **2.017** | * **0.023** |
| hsa-miR-214-5p | DOWN | 0.620 | 0.239 | DOWN | 0.629 | 0.334 |
| hsa-miR-218-5p | UP | 1.283 | 0.27 | DOWN | 0.725 | 0.433 |
| hsa-miR-224-5p | DOWN | 0.916 | 0.686 | UP | 1.191 | 0.499 |
| hsa-miR-23a-3p | DOWN | 0.735 | 0.156 | UP | 1.242 | 0.435 |
| **hsa-miR-24-3p** | **DOWN** | **0.417** | ** **0.002** | **DOWN** | **0.549** | * **0.014** |
| hsa-miR-34c-5p | UP | 1.483 | 0.095 | UP | 1.438 | 0.27 |
| hsa-miR-3529-3p | UP | 1.725 | 0.288 | UP | 2.670 | 0.16 |
| hsa-miR-378a-3p | DOWN | 0.956 | 0.878 | UP | 1.145 | 0.559 |
| hsa-miR-425-5p | UP | 1.020 | 0.953 | DOWN | 0.909 | 0.713 |
| **hsa-miR-99b-5p** | **DOWN** | **0.832** | **0.497** | **UP** | **1.567** | * **0.022** |

### Bootstrap correction

The data obtained from RT-qPCR was analyzed by a regression model using bootstrapping correction. The bootstrapping approach generated simulated datasets with the same statistical features of the assessed dataset, in order to better assess the significance of the variables included and the reproducibility of the models on new, different datasets.

### Method 1: PBP-M

By simulating up to 500 datasets with similar data distribution features and the given dataset, it was observed that the original-data AUC tended to converge at 80%, while the AUC of the simulated data returned a more normal distribution, with a median AUC -80-90%. Thus, the model based on the three microRNAs hsa-miR-24-3p, hsa-miR-200b-3p y hsa-miR-148b-3p is robust against data that fits within the given characteristics. Results are shown graphically in Fig. 1. It clearly indicated an improvement of the model, although AUC values for the 500 replication bootstrap approached were spread over the 0.8-1 range. The model was significantly predictive (p-value 0.003259), albeit its reproducibility in the low-to-mid range (R2 0.43, adjusted R2 0.36). Results are shown graphically in **Fig. 2**

### Method 2: PBP-N

By simulating up to 500 datasets with similar data distribution features and the given dataset, it was observed that the original-data AUC tended to converge at 80%, while the AUC of the simulated data returned a more normal distribution, with a median AUC -80-90%. Thus, the model based on the three microRNAs hsa-miR-24-3p, hsa-miR-200b-3p and hsa-miR-99b-5p is robust against data that fits within the given characteristics. Results are shown graphically in **Fig. 3****.** It clearly indicated an improvement of the model, although AUC values for the 500-replication bootstrap approached were spread over the 0.8-1 range. The model was significantly predictive (p-value 0.00023), albeit its reproducibility in the low-to-mid range (R2 0.4935, adjusted R2 0.4392). Results are shown graphically in **Fig. 4****.**

### Summary

The endometrial fluid obtained in a non-invasive way from 30 women who underwent in vitro fertilization (IVF) with frozen embryo transfer (FET) was analyzed. Of these, 15 women underwent successful implantation and 15 women did not. No significant differences were found between groups concerning these features. A commercial kit Invitrogen Total Exosome Reagent (PBP) was used to enrich the samples in extracellular vesicles before RNA extraction. Post EVs enrichment, the RNA extraction was performed using two different commercial kits mirVAna Paris kit (PBP-M) and Norgen kit (PBP-N).

A robust methodology for analyzing both vesicular and non-vesicular miRNAs from EF in clinical settings was established, where the sample is limited and no sophisticated equipment is available. Different methodologies have been evaluated comparing direct miRNAs extraction methods and methods with a previous step in EVs enrichment before the RNA extraction. The results showed that the protocols with a previous enrichment step of EVs obtained a higher miRNA detection.

The extracted RNA was analyzed by small RNA sequencing (SmallRNAseq). In the first phase ("Discovery"), the statistical analyses of the smallRNASeq results showed 16 miRNAs that were significantly differentiated between the two groups "receptive" (*implantative*) vs "non-receptive" (*non-implantative*). Thus, a number of miRNAs were selected from the *SmallRNAseq* "Discovery" results because they were found to be differentially expressed between the implantation (receptive endometrium) group and the non-implantation (non-receptive endometrium) group with consideration of the methods PBP-M, PBP-N or both and the results obtained with each method for each miRNA. In the second phase ("Validation"), these miRNAs were validated by RT-qPCR in the same samples using commercially available probes for these miRNAs. The results showed that a number of miRNAs where differentially expressed among the samples from women having a receptive endometrium and successful embryo implantation with respect to samples from women having a non-receptive endometrium and no embryo implantation. The data obtained from RT-qPCR was further confirmed by a regression model using bootstrapping correction.

Therefore, the present work provides evidence of the feasibility of a method for the reliable determination of endometrial receptivity based on the differential levels of miRNAs in samples from receptive and non-receptive endometria, specifically, by analysis of the levels of a miRNA signature a) comprising hsa-miR-24-3p, hsa-miR-200b-3p and/or hsa-miR-148b-3p and a miRNA signature b) comprising hsa-miR-24-3p, hsa-miR-200b-3p and/or hsa-miR-99b-5p.

## Claims

1. An *in vitro* method for determining endometrial receptivity in a female subject which comprises the steps of:
i) determining the level of the miRNAs from a miRNA signature, wherein said miRNA signature is selected from the group consisting of
- a miRNA signature a) comprising the hsa-miR-24-3p, hsa-miR-200b-3p and/or hsa-miR-148b-3p miRNAs, and
- a miRNA signature b) comprising the hsa-miR-24-3p, hsa-miR-200b-3p and/or hsa-miR-99b-5p miRNAs,
in at least one sample from said subject, and
ii) comparing the level of said miRNAs obtained in step (i) with a reference value for each of the miRNAs,
wherein a reduced level of hsa-miR-24-3p and/or hsa-miR-148b-3p, and/or an increased level of hsa-miR-200b-3p, and/or an increased level of hsa-miR-99b-5p, with respect to the reference value, is indicative that the endometrium is receptive, and
wherein an increased level of hsa-miR-24-3p and/or hsa-miR-148b-3p, and/or a reduced level of hsa-miR-200b-3p, and/or an increased level of hsa-miR-24-3p, and/or a reduced level of hsa-miR-200b-3p and/or hsa-miR-99b-5p, with respect to the reference value, is indicative that the endometrium is not receptive.

2. The method according to claim 1, wherein
signature a) comprises the hsa-miR-24-3p, hsa-miR-200b-3p and hsa-miR-148b-3p miRNAs; and
signature b) comprises the hsa-miR-24-3p, hsa-miR-200b-3p and hsa-miR-99b-5p miRNAs, and
wherein a reduced level of hsa-miR-24-3p and hsa-miR-148b-3p, and an increased level of hsa-miR-200b-3p, and/or a reduced level of has-miR-24-3p and an increased level of has-miR-200b-3p and hsa-miR-99b-5p, with respect to the reference value, is indicative that the endometrium is receptive, and
wherein an increased level of hsa-miR-24-3p and hsa-miR-148b-3p, and a reduced level of hsa-miR-200b-3p, and/or an increased level of has-miR-24-3p and a reduced level of has-miR-200b-3p and hsa-miR-99b-5p, with respect to the reference value, is indicative that the endometrium is not receptive.

3. An *in vitro* method for selecting a female subject as a candidate to receive a therapy to increase or restore endometrial receptivity which comprises
a. determining the endometrial receptivity of said subject by following the method according to claim 1, and
b. selecting a subject as a candidate to receive an adequate therapy to increase or restore endometrial receptivity if the endometrium of the subject is not receptive.

4. The method according to any one of claims 1 to 3, wherein the levels of the miRNAs are indicative of receptivity within the same *in vitro* fertilization cycle in which the sample from the subject has been obtained or wherein the subject is selected as candidate to receive an adequate therapy to increase or restore endometrial receptivity within the same *in vitro* fertilization cycle in which the sample from the subject has been obtained.

5. The method according to any one of claims 1 to 4, wherein the sample is endometrial fluid.

6. The method according to claim 5, wherein said sample of endometrial fluid comprises free and extracellular vesicle-associated miRNAs.

7. The method according to any one of claims 1 to 6, wherein prior to step i) said method comprises a non-invasive step of collecting at least one sample of endometrial fluid comprising free and extracellular vesicle-associated miRNAs.

8. The method according to any one of claims 5 to 7 wherein the sample of endometrial fluid comprising free and extracellular vesicle-associated miRNAs is subjected to a step of enrichment in extracellular vesicles prior to the determination of the levels of the miRNAs.

9. The method according to any one of claims 1 to 8, wherein the reference value for each miRNA is the level of said miRNA determined in a sample of endometrial fluid from a receptive endometrium.

10. The method according to any one of claims 1 to 9, wherein the levels of the miRNAs obtained in step (i) are normalized using the levels of at least one additional miRNA.

11. The method according to claim 10, wherein the normalization is carried out using the levels of hsa-miR-200c-3p, of hsa-miR-92a-3p or of a combination thereof.

12. The method according to any one of claims 1 to 11, wherein the female subject is undergoing an *in vitro* fertilization procedure.

13. A kit comprising reagents adequate for the determination of the level of miRNAs forming part of a miRNA signature selected from the group consisting of a miRNA signature consisting of the hsa-miR-24-3p, hsa-miR-200b-3p and hsa-miR-148b-3p miRNAs, and a miRNA signature consisting of the hsa-miR-24-3p, hsa-miR-200b-3p and hsa-miR-99b-5p miRNAs.

14. The kit according to claim 13, wherein the reagents adequate for the determination of the level of said one or more miRNAs are oligonucleotides that hybridize specifically to said one or more miRNAs.

15. Use of a kit according to any one of claims 13 or 14 for determining endometrial receptivity, or for identifying a woman as a candidate to receive a therapy to increase endometrial receptivity.
